# EUROPEAN PATENT APPLICATION

(11) **EP 4 778 942 A1**
(43) Date of publication of application: **22.07.2026**
(21) Application number: 23951909.3
(22) Date of filing: 15.09.2023
(51) Int. Cl.: C07K 19/00, C12N 15/62, A61K 39/145, A61K 39/12, A61P 31/16

(54) **BROAD-SPECTRUM INFLUENZA A VACCINE IMMUNOGEN COMPOSITION AND USE THEREOF**

(71) Applicant: Fudan University, Shanghai 200433 (CN)
(72) Inventor: XU, Jianqing, Shanghai 200433 (CN); ZHANG, Xiaoyan, Shanghai 200433 (CN); YANG, Tianhan, Shanghai 200433 (CN)
(74) Representative: Pfenning, Meinig & Partner mbB
(86) International application number: PCT/CN2023/119171
(87) International publication number: WO 2025/054979

(57) **Abstract**

A broad-spectrum influenza A vaccine immunogen composition and the use thereof. Provided is a nucleic acid molecule encoding an immunogenic peptide, the immunogenic peptide comprising: tandem M2e peptide segments, an HA2 peptide segment derived from influenza virus H7N9 subtype; a multimerized motif peptide segment, and optionally, one or more linker peptide segments independently located between the peptide segments, the nucleic acid molecule comprising nucleic acids encoding the peptide segments. The broad-spectrum influenza A vaccine can induce organisms to generate antigen-specific immune responses, and can generate high-titer antibodies in as early as 14 days after the last vaccination, which can completely protect against challenge with various influenza A virus subtypes.

## Description

### TECHNICAL FIELD

The present application belongs to the field of vaccines and biotechnology. Specifically, the present application relates to a broad-spectrum influenza A vaccine immunogen composition and use thereof.

### BACKGROUND

Influenza is an acute infectious disease caused by the influenza virus, transmitted through the respiratory tract. The influenza virus is an antisense single-stranded RNA virus belonging to the *Orthomyxoviridae* family, with a genome comprising 8 gene segments. Based on the nucleoprotein (NP) and matrix protein (M) of the influenza virus, it can be divided into four types: A, B, C, and D. Influenza A viruses can infect humans, avian, swine, and other organisms, causing diseases, and it commonly circulates seasonally in human populations and can potentially cause pandemics. Influenza B viruses only infect humans and generally cause seasonal and regional epidemics. Influenza C virus infection generally causes only mild symptoms or is asymptomatic. Influenza D virus generally does not infect humans. Influenza A viruses can be further divided into multiple subtypes based on their surface hemagglutinin protein (HA) and neuraminidase (NA). Recombination can occur between these subtypes, producing new subtypes or strains. Furthermore, due to the low fidelity of the replicase, influenza virus is prone to mutation during replication, also producing new strains. Influenza B is mainly divided into two lineages based on its HA protein: the Victoria lineage and the Yamagata lineage.

Worldwide, seasonal influenza infections cause 3 to 5 million cases of severe symptoms and 290,000 to 650,000 deaths annually. Influenza viruses are transmitted through the respiratory tract and can cause clinical manifestations such as high fever, body aches, fatigue, and cough, which can be life-threatening in severe cases. Currently, the main strains causing seasonal influenza in humans are H1N1, H3N2, and influenza B viruses.

Vaccination is the primary way to prevent influenza virus infection. Currently available influenza vaccines, in terms of vaccine strain composition, include trivalent and quadrivalent vaccines. Trivalent vaccines include H1N1, H3N2, and one lineage from Influenza B, while quadrivalent vaccines include H1N1, H3N2, and two lineages from Influenza B. In terms of manufacturing processes, split vaccines, recombinant protein vaccines, and live attenuated vaccines are mainly included. The World Health Organization (WHO) predicts and publishes the circulating strains in the Northern and Southern Hemispheres annually for the current influenza season, and all influenza vaccine manufacturing must utilize WHO-recommended vaccine strains or sequences. Due to the inaccuracy of predictions, when the predicted vaccine strain differs from the circulating strain, the protective efficacy of the influenza vaccine may be less than 20%. Because of the rapid changes in circulating strains, current influenza vaccines require annual vaccination, increasing costs and reducing patient adherence. Overcoming the high mutation rate of the influenza virus is the core issue in influenza vaccine development.

The potency of commercially available influenza vaccines is typically measured using a Hemagglutination Inhibition (HI) assay, which primarily evaluates the ability of serum after vaccination to inhibit hemagglutination of the influenza virus HA protein. And the titer of hemagglutination inhibition antibodies reflects the protective efficacy provided by the vaccine. However, most hemagglutination protective antibodies target the head of the HA protein, which has a high mutation rate. Once a mutation occurs, the original protective antibodies become ineffective, thus rendering the vaccine ineffective.

To overcome the problem of vaccine ineffectiveness due to high mutation rates in influenza, vaccines need to be able to induce immunity against conserved portions of the influenza virus to provide broad-spectrum protection. Compared to the highly mutated head region, the HA protein stem region is highly conserved and is a good candidate immunogen region. Furthermore, the extracellular domain of the influenza virus M2 protein (M2e) is highly conserved across multiple influenza subtypes, also making it a good candidate immunogen region. However, both the HA stem region and the M2e protein have low immunogenicity, and current vaccination or natural infection is unlikely to induce high-titer immune responses. Developing a broad-spectrum influenza vaccine targeting these two regions requires improving the immunogenicity of the HA stem region and the M2e protein.

mRNA (messenger ribonucleic acid) vaccines are an emerging vaccine technology that has achieved good results in the development of vaccines against the novel coronavirus, with several companies successfully launching their own mRNA vaccines for COVID-19. mRNA vaccines are produced without relying on cells, but rather through *in vitro* transcription synthesis. Due to its design and process advantages, only the sequence needs to be obtained for design and production, allowing mRNA vaccine production to skip the traditional strain/cell line culture and amplification process, simplifying the production flow and enabling large-scale production in a short time. Furthermore, because the entire production process is virus-free, and *in vitro* transcription and purification are cell-free, there are no biosafety risks such as virus leakage, and there is no risk of residual host cell nucleic acid or protein in the finished vaccine. The mRNA production process is easy to scale up rapidly, and the vaccines are safe and effective, possessing high application value.

T-cell immune responses play a crucial role in clearing viral infections, exhibiting a rapid response rate in immune responses induced by secondary exposure to immunogens. When the body has pre-existing T-cell immunity, upon encountering pathogen infection, T cells can rapidly generate a response, proliferate quickly, and thus rapidly control the infection. Furthermore, T- cell immune responses also play an important role in clearing pathogens from long-term infections.

In summary, the technical challenges of developing a broad-spectrum and highly effective influenza vaccine based on conserved immunogen components of influenza that can induce a strong immune response and has advantages such as rapid production urgently need to be addressed.

### SUMMARY OF THE INVENTION

The present disclosure addresses the problems existing in the prior art by providing an mRNA molecule that encodes the stem region (HA2) of the influenza hemagglutinin protein and the extracellular region of the M2 protein (M2e), which are linked to multimerized motifs. The present mRNA can efficiently express the hemagglutinin protein stem region (HA2) of the H7N9 subtype and the extracellular region of the M2 protein (M2e), which then form multimers, which can be used for broad-spectrum prevention of influenza A after immunization. Based on the mRNA, the present application further provides a composite mRNA comprising said mRNA which is linked to other components (such as T-cell antigens, interleukin IL-21, etc.) using linking elements.

In some aspects of the present application, a nucleic acid molecule encoding an immunogenic peptide is provided, wherein the immunogenic peptide comprises (a) a tandem M2 protein extracellular domain (M2e) peptide segment; (b) a hemagglutinin protein stem extracellular domain (HA2) peptide segment derived from an H7N9 influenza virus subtype; (c) a multimerized motif peptide segment; (d) optionally, one or more linker peptide segments independently located between the peptide segments; and (e) optionally, one or more other peptide segments, such as a T-cell antigen peptide segment; and wherein the nucleic acid molecule comprises nucleic acids encoding each said peptide segment.

In some aspects of the present application, an immunogenic peptide is provided comprising (a) tandem M2 protein extracellular domain (M2e) peptide segment; (b) a hemagglutinin protein stem extracellular domain (HA2) peptide segment derived from an H7N9 influenza virus subtype; (c) a multimerized motif peptide segment; (d) optionally, one or more linker peptide segments independently located between the peptide segments; and (e) optionally, one or more other peptide segments, such as a T-cell antigen peptide segment. In some embodiments, the immunogenic peptide is encoded by a nucleic acid molecule of the present application.

In some aspects of the present application, a vector or host cell is provided comprising the nucleic acid molecules of the present application.

In some aspects of the present application, an influenza vaccine is provided comprising (i) one or more nucleic acid molecules, immunogenic peptides and/or vectors of the present application; and (ii) an immunologically acceptable carrier, delivery system or adjuvant.

In some aspects of the present application, the use of the nucleic acid molecules, immunogenic peptides, vectors and/or influenza vaccines of the present application in the preparation of products for the prevention and/or treatment of influenza is provided.

In some aspects of the present application, a method for preventing and/or treating influenza A is provided, wherein the method comprises administering a subject in need thereof a product comprising the nucleic acid molecule, immunogenic peptide, vector and/or influenza vaccine of the present application.

In some aspects of the present application, nucleic acid molecules, immunogenic peptides, vectors and/or influenza vaccines of the present application are provided for use in the prevention and/or treatment of influenza A.

Those skilled in the art may arbitrarily combine the above technical solutions and technical features without departing from the inventive concept and the scope of protection of the present disclosure. Other aspects of the present disclosure are obvious to those skilled in the art based on the disclosure of present application.

### DESCRIPTION OF FIGURES

The present disclosure will be further described below with reference to the accompanying figures. These figures are only for illustrating the embodiments of the present disclosure and are not intended to limit the scope of the disclosure.

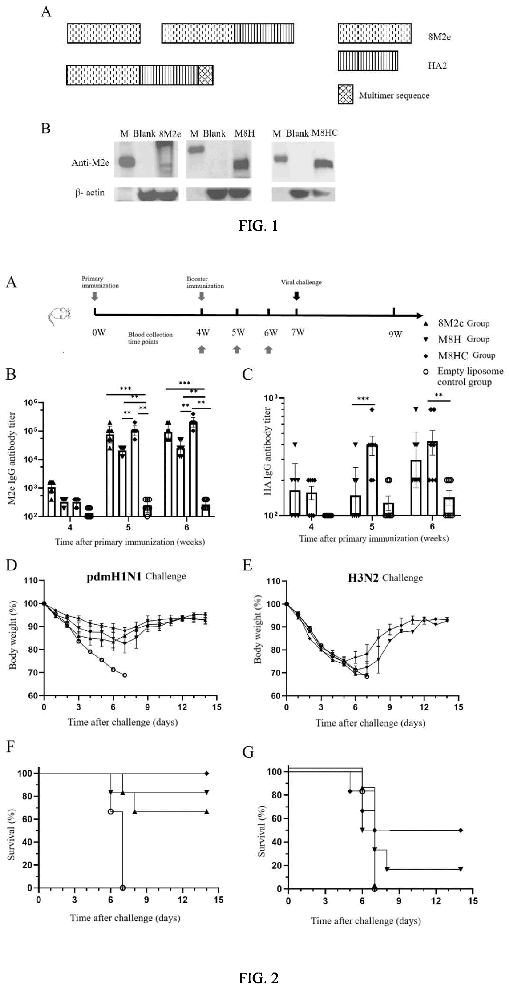

**Fig. 1** shows a schematic diagram of the construction of immunogens comprising 8 tandem repeats of M2e (8M2e), 8M2e-HA (M8H), or 8M2e-HA-collagen (M8HC) (A); and the expression verification in HEK293 cells transfected with *in vitro* transcribed mRNA (B).
**Fig. 2** shows the mouse immunization protocol (A); the humoral immune response levels against M2e protein (B) and H7N9 HA protein (C) in mice after primary immunization (i.e., "first-dose immunization" in the figure) and booster immunization with mRNA expressing 8M2e, M8H, or M8HC; and the body weight (D, E) and survival (F, G) of mice infected with lethal influenza H1N1 (pdmH1N1: A/California/04/2009) and H3N2.

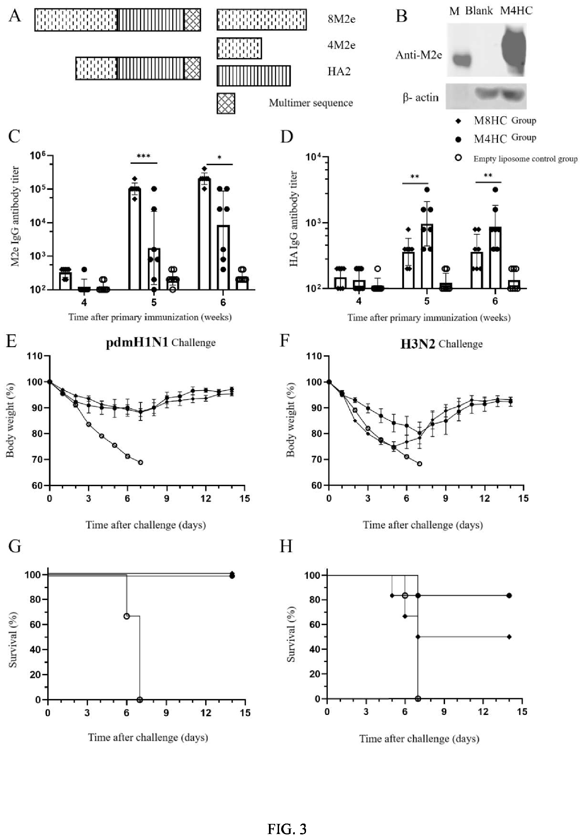

**Fig. 3** shows the schematic diagram of the construction of immunogen 8M2e-HA-collagen (M8HC) or 4M2e-HA-collagen (M4HC) (A); the expression verification of mRNA-M4HC transfected HEK293 cells (B); the humoral immune response levels against M2e protein (C) and H7N9 HA protein (D) in mice after primary and booster immunization with mRNA expressing M8HC or M4HC; as well as the body weight (E, F) and survival (G, H) of mice infected with lethal influenza H1N1 (A/California/04/2009) and H3N2.

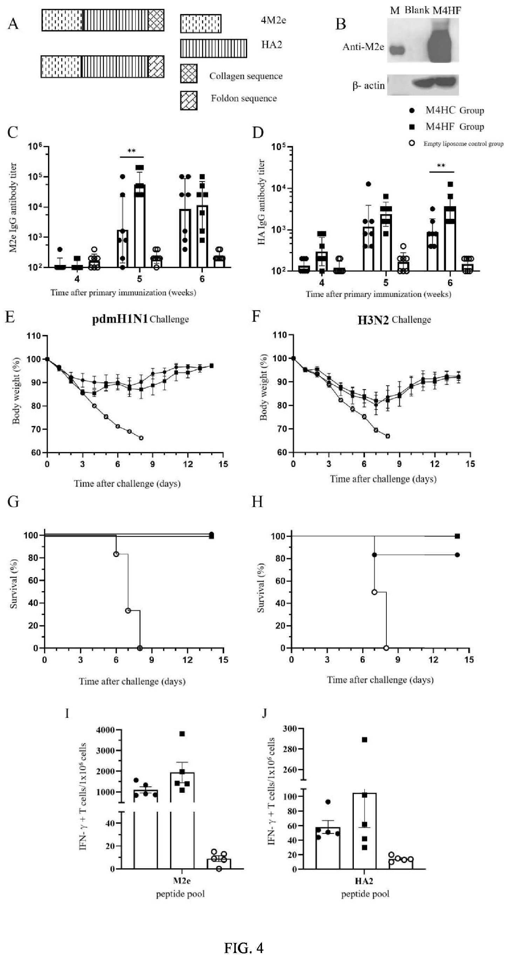

**Fig. 4** shows the schematic diagram of the construction of immunogen 4M2e-HA-collagen (M4HC) or 4M2e-HA-foldon (M4HF) (A); expression verification of mRNA-M4HF transfected HEK293 cells (B); humoral immune response levels against M2e protein (C) and H7N9 HA protein (D) in mice after primary and booster immunization with mRNA expressing M4HC or M4HF; body weight (E, F) and survival (G, H) of mice after infection with a lethal dose of influenza H1N1 (A/California/04/2009) and H3N2 infection; and the response of T cells to M2e and HA2 peptide libraries (I, J) 10 days after booster immunization in mice.

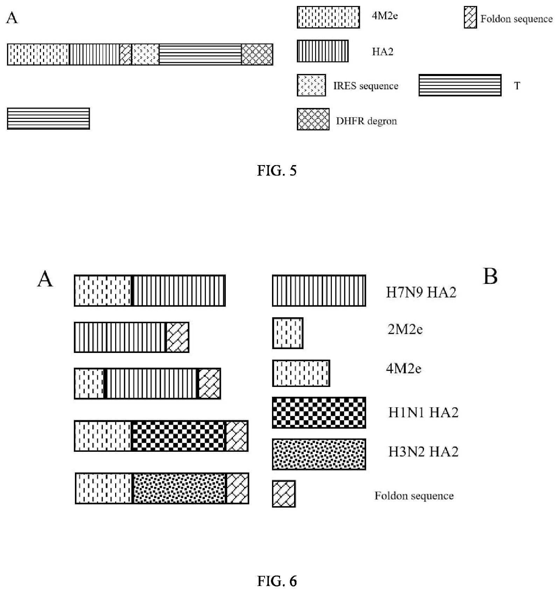

**Fig. 5** shows a schematic diagram of the construction of immunogen 4M2e-HA-foldon-IRES-T (M4HF-T) and T (A).
**Fig. 6** shows schematic diagrams of the construction of immunogens containing only 4M2e and H7N9 HA2 (M4H), containing only H7N9 HA2 and multimer sequence (HF), 2M2e-HA-foldon (M2HF) with the four repeating M2e in M4HF reduced to two, and 4M2e-H1N1 HA-foldon (M4H1F) and 4M2e-H3N2 HA-foldon (M4H3F) formed by replacing the HA2 from H7N9 in the design of M4HF with HA2 from H1N1 or H3N2 respectively.

### DETAILED DESCRIPTION

The present disclosure relates to the field of vaccines, and in particular to an mRNA vaccine that induces a broad-spectrum immune response against influenza A virus and application thereof.

All numerical ranges provided herein are intended to clearly include all values falling between the range endpoints and the numerical ranges therebetween. Features mentioned in the present disclosure or in the embodiments may be combined. All features disclosed in the present application may be used in combination with any composition form, and each feature disclosed in the present application may be replaced by any alternative feature that can provide the same, equivalent or similar purpose. Unless otherwise specified, the disclosed features are only general examples of equivalent or similar features.

As used herein, the term "about" in the context of a number or range means ±10% of the number or range referenced or claimed.

It should be understood that when a parameter range is provided, the present invention also provides all integers within that range and values to the first decimal place. For example,"0.1-2.5 mg/day" includes 0.1 mg/day,0.2 mg/day,0.3 mg/day, *etc.,* up to 2.5 mg/day.

As used herein, "comprising", "having", or "including" encompasses "consisting of", "consisting mainly of", "consisting essentially/substantially of", and "be comprised of"; "consisting mainly of", "consisting essentially/substantially of", and "consists of" are sub-concepts of "comprising", "having", or "including".

To address the aforementioned technical problems in this field, the present disclosure provides a nucleic acid molecule encoding a hemagglutinin protein stem region (HA2) derived from the H7N9 subtype (e.g., A/Shanghai/02/2013 strain) and multiple tandem M2 protein extracellular regions (M2e), which are linked to different multimerized motifs (e.g., collagen/foldon/honeybee melittin/tetrabrachion helical peptide/GCN4). The nucleic acid molecule can be used to efficiently express the corresponding chimeric immunogenic peptide. For example, the nucleic acid molecule can be loaded into an mRNA vector, allowing the mRNA to efficiently express the H7N9 subtype hemagglutinin protein stem region (HA2) and multiple tandem M2 protein extracellular regions (M2e) and form multimers. The mRNA can be delivered using a suitable delivery vector (e.g., encapsulated in liposome nanoparticles) for immunization to prevent or treat infection with various influenza A virus subtypes. Alternatively, the chimeric immunogenic peptide of the present application can be directly administered to the recipient.

Based on the aforementioned technical solutions, the present application further provides an improved solution, wherein an mRNA is provided, which encodes the hemagglutinin protein stem region (HA2) containing the H7N9 subtype (A/Shanghai/02/2013 strain) and the extracellular region of the M2 protein (M2e), linked to different multimerized motifs (collagen/foldon/honeybee melittin/tetrabrachion helical peptide/GCN4), and further linked to T-cell antigens or cytokines. The mRNA is finally loaded into an mRNA vector, or the M2e-HA-polymerizing sequence and the T-cell antigen are loaded separately. The mRNA, upon entering cells, can efficiently express the H7N9 subtype hemagglutinin protein stem region (HA2) and the extracellular region of the M2 protein (M2e), form multimers, and simultaneously express T-cell antigens or cytokines. The mRNA can be delivered using a suitable delivery vector (e.g., encapsulated in liposome nanoparticles) for immunization to prevent or treat infection with various subtypes of influenza A virus. Alternatively, the chimeric immunogenic peptide of the present application can be directly administered to the recipient.

The broad-spectrum influenza A vaccine designed in the present disclosure induces high-titer protective antibodies 7 days after completion of the full vaccination course (e.g., intramuscular injection). Three weeks after completion of the full vaccination course, mice challenged with a lethal dose of the virus showed that the vaccine-induced immune response completely resisted lethal doses of H1N1 and H3N2 viruses. The broad-spectrum influenza A vaccine of the present disclosure (e.g., an RNA vaccine) induces effective protective antibodies after two doses of immunization. This broad-spectrum influenza A vaccine exhibits good immunogenicity and forms a strong immune protection after immunization. It also has low production costs, simple preparation techniques, and is suitable for rapid, efficient, and large-scale vaccine preparation, showing great development potential. Furthermore, the introduction of T-cell antigens in the improved solution can induce mice to produce specific T cells capable of responding to multiple influenza viruses and secreting cytokines.
Three weeks after completion of the full vaccination course, mice challenged with a lethal dose of the virus showed that the vaccine-induced humoral and cellular immune responses enabled the mice to survive lethal doses of H1N1 and H3N2 viruses. The RNA vaccine of the present disclosure can induce effective humoral and cellular immunity after two doses, which is beneficial for the rapid clearance of influenza A virus.

### Immunogenic peptides and encoding molecules thereof

As used herein, the term "immunogenic peptide" refers to a peptide capable of inducing an immune response against a variety of influenza A viruses, comprising the following components: (a) a tandem M2 protein extracellular domain (M2e) peptide segment; (b) a hemagglutinin protein stem extracellular domain (HA2) peptide segment derived from the H7N9 influenza virus subtype; (c) a multimerized motif peptide segment; (d) optionally, one or more linker peptide segments located independently between the peptide segments; and (e) optionally, one or more other peptide segments, such as T-cell antigen peptide segments.

As used herein, the term "link" has a broad meaning and can include various forms such as fusion, covalent connection, and coupling.

When referring to sequence identity or homology in describing or defining an amino acid sequence or nucleotide sequence, the amino acid sequence or nucleotide sequence has a homology or sequence identity of 80% or higher, 85% or higher, 90% or higher, 95 % or higher, 96% or higher, 97% or higher, 98% or higher, or 99% or higher compared to the reference sequence.

The immunogenic peptides may also include their variant forms, such as the deletion, insertion, and/or substitution of one or more amino acids (typically 1-50, preferably 1-30, more preferably 1-20, most preferably 1-10, e.g., 1, 2, 3, 4, 5, 6, 7, 8, 9, or 10), as well as the addition of one or more amino acids (typically up to 20, preferably up to 10, more preferably up to 5) at the C-terminus and/or N-terminus. For example, in the art, substitution with amino acids of similar properties typically does not change the function of the protein or polypeptide. Similarly, the addition of one or more amino acids at the C- terminus and/or N- terminus typically does not alter the function of the protein or peptide.

The immunogenic peptides can be produced through recombinant expression under appropriate environment and conditions, such as by the encoding nucleic acid molecules, vectors, or host cells of the present disclosure; they can also be obtained through chemical synthesis, as long as they have the required amino acid sequence, immunogenicity, and reactivity.

As used herein, the terms "immunogenic peptide-encoding molecule" and "coding sequence" are used interchangeably and refer to the nucleic acid molecule encoding the immunogenic peptide described herein.

In some embodiments, the nucleic acid molecule may be selected from, for example: (i) nucleotide molecules having the nucleotide sequences set forth in SEQ ID NO: 26, SEQ ID NO: 28, SEQ ID NO: 34, SEQ ID NO: 40, and SEQ ID NO: 42; (ii) molecules that hybridize with (i) under stringent conditions; (iii) nucleotide molecules that are homologous to or sequence-similar to (i) and capable of expressing functional immunogenic peptides, for example, wherein each segment of the nucleotide molecule has a sequence identity or homology greater than or equal to 80%, greater than or equal to 85%, greater than or equal to 90%, greater than or equal to 95%, greater than or equal to 96%, greater than or equal to 97%, greater than or equal to 98%, and greater than or equal to 99% with nucleotide sequences shown in SEQ ID NO: 26, SEQ ID NO: 28, SEQ ID NO: 34, SEQ ID NO: 40, and SEQ ID NO: 42, respectively; or has a sequence identity or homology greater than or equal to 80%, greater than or equal to 85%, greater than or equal to 90%, greater than or equal to 95%, greater than or equal to 96%, greater than or equal to 97%, greater than or equal to 98%, and greater than or equal to 99% with sequences in (i) or (ii) and is capable of expressing a functional immunogenic peptide; (iv) nucleic acid molecules that have one or more nucleotide substitutions, deletions, or additions in the nucleotide sequence defined in (i) or (ii) and are capable of expressing a functional immunogenic peptide.

As used herein, the term " strict conditions " refers to (1) hybridization and elution at lower ionic strength and higher temperature, such as 0.2×SSC, 0.1% SDS, 60°C; or (2) hybridization with a denaturing agent, such as 50% (v/v) formamide, 0.1% bovine calf serum /0.1% Ficoll, 42°C, etc.; or (3) hybridization occurs only when the identity between the two sequences is at least 50%, preferably 55% or more, 60% or more, 65% or more, 70% or more, 75% or more, 80% or more, 85% or more, or 90% or more, and more preferably 95% or more.

The full-length nucleotide sequences or fragments thereof disclosed herein can generally be obtained by PCR amplification, recombinant methods, or artificial synthesis. For PCR amplification, primers can be designed based on the nucleotide sequences disclosed herein, and commercially available cDNA libraries or cDNA libraries prepared using conventional methods known to those skilled in the art can be used as templates to amplify the relevant sequences. When the sequences are long, two or more PCR amplifications are often required, and then the fragments amplified from each amplification are spliced together in the correct order.

In some embodiments, the M2e peptide segment in the immunogenic peptide is derived from one or more influenza virus strains selected from the group consisting of H1N1, H2N2, H3N2, H3N8, H5N1, H5N2, H5N6, H7N1, H7N2, H7N3, H7N7, H7N9, H9N2, H10N8, and H11N2 subtypes. In some embodiments, the M2e peptide segment is derived from influenza virus strains whose hosts are animals selected from the group consisting of: mammalian (e.g., human, non-human primates, swine, horse, cattle, sheep, bat, sea lion, seal, rat, mouse, ferret), avian (e.g., domestic poultry or wild birds), or zoonotic (e.g., human-animal, human-avian, or swine-avian) influenza virus strains. In some embodiments, each of the M2e regions in the M2e peptide segment is derived from the same or different sources.

In some embodiments, the M2e peptide segment comprises 2-12 tandem M2e regions, preferably 4-8 tandem M2e regions, such as 4, 5, 6, 7, 8, 9, 10, 11, or 12 tandem M2e regions. In some embodiments, each of the M2e regions in the M2e peptide segment is directly linked or linked via a linker, for example, the linker is independently selected from flexible linkers, rigid linkers, and cleavable linkers, such as AAA, (G4S)ₙ, e.g., (G4S)₃, GSAGSAAGSGEF, (Gly)ₙ, (EAAAK)ₙ, EFPKPSTPPGSSGGAP, KESGSVSSEQLAQFRSLD, EGKSSGSGSESKST, where n is an integer between 2 and 10, e.g., n=3. In some embodiments, the linker comprises one or more repeating fragments selected from those set forth in SEQ ID NOs: 45-50.

In some embodiments, the M2e region has the amino acid sequence set forth in SEQ ID NO: 13, SEQ ID NO: 15, SEQ ID NO: 17, or SEQ ID NO: 19, or an amino acid sequence having at least 80% sequence identity with said amino acid sequences. In some embodiments, the nucleic acid encoding the M2e region has the nucleotide sequences set forth in SEQ ID NO: 14, SEQ ID NO: 16, SEQ ID NO: 18, and SEQ ID NO: 20, or a nucleotide sequence having at least 80% sequence identity with said nucleotide sequences, e.g., sequences obtained by codon optimization or substitution of one or more nucleotides from the aforementioned nucleotide sequences.

In some embodiments, the M2e peptide segment has the amino acid sequence set forth in SEQ ID NO: 21 or SEQ ID NO: 23, or an amino acid sequence having at least 80% sequence identity with said amino acid sequence. In some embodiments, the nucleic acid encoding the M2e peptide segment has the nucleotide sequence set forth in SEQ ID NO: 22 or SEQ ID NO: 24, or a nucleotide sequence having at least 80% sequence identity with said nucleotide sequence, e.g., a sequence obtained by codon optimization or substitution of one or more nucleotides of the aforementioned nucleotide sequence.

In some embodiments, the HA2 peptide segment is derived from the H7N9 subtype strain, such as A/Shanghai/02/2013 strain or A/Anhui/01/2013 strain. In some embodiments, the HA2 peptide segment comprises one or more mutations compared to the native HA2 peptide segment, such as an N82 mutation at amino acid position 82, preferably N82R, N82K, N82D, or N82E; and an N154 mutation at position 154, preferably N154H. In some embodiments, the HA2 peptide segment is linked to a partial fragment of HA1, for example, comprising an HA2 peptide segment that anchors the HA protein to the stem of the viral lipid envelope, and a partial HA1 peptide segment. In some embodiments, the HA2 peptide segment has the amino acid sequence set forth in SEQ ID NO: 1, or an amino acid sequence having at least 80% sequence identity with said amino acid sequence. In some embodiments, the nucleic acid encoding the HA2 peptide segment has the nucleotide sequence set forth in SEQ ID NO: 2, or a nucleotide sequence having at least 80% sequence identity with said nucleotide sequence, e.g., a sequence obtained by codon optimization or substitution of one or more nucleotides of the aforementioned nucleotide sequence.

In some embodiments, the multimerized motif peptide segment is selected from collagen, the trimerized motif Foldon of T4 phage fibrin, the tetrabrachion helical peptide, and GCN. In some embodiments, the multimerized motif peptide segment has the amino acid sequence set forth in SEQ ID NO: 3, SEQ ID NO: 5, SEQ ID NO: 7, and SEQ ID NO: 9, or an amino acid sequence having at least 80% sequence identity with said amino acid sequence. In some embodiments, the nucleic acid encoding the multimerized motif peptide segment has the nucleotide sequence set forth in SEQ ID NO: 4, SEQ ID NO: 6, SEQ ID NO: 8, and SEQ ID NO: 10, or a nucleotide sequence having at least 80% sequence identity with said nucleotide sequence, e.g., a sequence obtained by codon optimization or substitution of one or more nucleotides from the aforementioned nucleotide sequence.

In some embodiments, the immunogenic peptide further comprises (e) one or more other peptide segments. In some embodiments, the other peptide segments include one or more peptide segments selected from the group consisting of T-cell antigen peptides, transferrin, FF peptides, cyclic peptide nanotubes, and IL-21.

In some embodiments, the immunogenic peptide may comprise other portions that are further linked, for example, to enhance the stability of the immunogenic stem or fusion protein, improving neutralizing antibody responses, forming multimers, increasing cellular responses, etc. In some embodiments, the other portions may include, but are not limited to viral or host-derived proteins, T-cell antigens, transferrin (Fn), IL-2, IL-7, IL-12, IL-18, IL-21, GM-CSF, CD40L, CD40-stimulating antibodies, PD-1 and PD-L1 antibodies, CTLA4 antibodies, chemokines CXCL9, CXCL10, CXCL11, CXCL12, CXCL3, XCL1, CCL4, CCL20, cholera toxin and subunits thereof, bacterial flagellin, FimH, etc.

In some embodiments, the T-cell antigen comprises an amino acid sequence set forth in SEQ ID NO: 11, or is encoded by a nucleic acid molecule of a nucleotide sequence set forth in SEQ ID NO: 12. In some embodiments, the T-cell antigen is as disclosed in Chinese Patent Application No. 201880001963.X (Publication No. CN 111315407A), for example, the T-cell antigen has the sequence set forth in SEQ ID NO: 1 or 2 of that application (i.e., the sequences set forth in SEQ ID NOs: 51 and 52 in the present application). In some embodiments, the T-cell antigen is obtained using the method disclosed in Chinese Patent Application No. 201880001963.X (Publication No. CN 111315407A). In some embodiments, the obtained T-cell antigen undergoes sequence optimization.

In some embodiments, the immunogenic peptide may further comprise elements such as signal peptides, linkers, and molecular tags. For example, a signal peptide element may refer to an amino acid sequence that guides protein secretion, localization, and/or delivery, and its length is typically 5-30 amino acids. In some embodiments, the signal peptide element may be selected from native signal peptides of the proteins, CD33 protein signal peptides, CD8 protein signal peptides, CD16 protein signal peptides, mouse IgG1 antibody signal peptides, and influenza HA protein signal peptides. For example, a linker peptide sequence (or linker) may refer to a short peptide that serves to link different elements in the fusion protein described herein, and its length is typically 1-50 (e.g., 5-50, 5-40, 10-40) amino acids. Generally, linker peptides do not affect or significantly affect the correct folding and spatial conformation of the peptides of the present disclosure. In some embodiments, the linker peptide element may be selected from (G4S)₃ linker, (G4S)ₙ, GSAGSAAGSGEF, (Gly)₆, EFPKPSTPPGSSGGAP, KESGSVSSEQLAQFRSLD, (_{Gly)8}, EGKSSGSGSESKST.

In some embodiments, other peptide segments include a T-cell antigen peptide segment having the amino acid sequence set forth in SEQ ID NO: 11, or an amino acid sequence having at least 80% sequence identity with said amino acid sequence.

In some embodiments, the nucleic acid encoding the T-cell antigen peptide segment has the nucleotide sequence set forth in SEQ ID NO: 12, or a nucleotide sequence having at least 80% sequence identity with the nucleotide sequence, e.g., a sequence obtained by codon optimization or substitution of one or more nucleotides from the aforementioned nucleotide sequence. In some embodiments, the T-cell antigen peptide segment is linked to a linking element, for example, the linking element is selected from IRES, T2A, and P2A. In some embodiments, the T-cell antigen peptide segment is further linked with a degron, such as the degron DHFR.

In some embodiments, peptide segments of the immunogenic peptide are independently linked directly or via a linker, for example, the linker is independently selected from: flexible linkers, rigid linkers and cleavable linkers, such as AAA, (G4S)ₙ (e.g. (G4S)₃), GSAGSAAGSGEF, (Gly)ₙ, (EAAAK)ₙ, EFPKPSTPPGSSGGAP, KESGSVSSEQLAQFRSLD, EGKSSGSGSESKST, where n is an integer between 2 and 10 (e.g., n=3).

In some embodiments, the immunogenic peptide has the amino acid sequences set forth in SEQ ID NO: 25, SEQ ID NO: 27, SEQ ID NO: 33, and SEQ ID NO: 39, or an amino acid sequence having at least 80% sequence identity with said amino acid sequences.

In some embodiments, the nucleic acid encoding the immunogenic peptide has the nucleotide sequence set forth in SEQ ID NO: 26, SEQ ID NO: 28, SEQ ID NO: 34, SEQ ID NO: 40, SEQ ID NO: 42, or a nucleotide sequence having at least 80% sequence identity with said nucleotide sequence, e.g., a sequence obtained by codon optimization or substitution of one or more nucleotides of the aforementioned nucleotide sequence.

In some embodiments, the nucleic acid molecule is a DNA molecule or an RNA molecule. In some embodiments, the nucleic acid molecule is an mRNA molecule, such as a self-amplifying mRNA (SAM) molecule or a non-replicating mRNA molecule, preferably a SAM
molecule; wherein, when the mRNA molecule is a non-replicating mRNA molecule, it further includes a 5'-cap structure and a 3'-poly(A) tail.

### Vector and host cell

The present disclosure also relates to a vector containing a nucleic acid molecule encoding the immunogenic peptide of the present application, and/or a host cell generated by genetic engineering using the vector. When the constructed nucleic acid molecule is mRNA, the mRNA can be applied directly without a host cell.

In the production of recombinant immunogenic peptides using host cells, the encoding sequences disclosed herein can be used to express or produce recombinant immunogenic peptides via conventional recombinant DNA technology (Science, 1984; 224: 1431). Generally, the following steps are involved:
(1) Transforming the encoding nucleotide molecule of the present disclosure, or a recombinant expression vector comprising a nucleotide molecule, into a suitable host cell;
(2) Culturing the host cells in a suitable culture medium;
(3) Isolating and purifying proteins or polypeptides from culture media or cells.

In the present disclosure, the term "vector" refers to bacterial plasmids, bacteriophages, yeast plasmids, animal cell viruses, mammalian cell viruses, or other vectors well known in the art. Vectors may be selected from mRNA vectors, DNA plasmid vectors, recombinant viral vectors, and recombinant bacterial vectors; wherein the mRNA vector is selected from linear, circular, and self-replicating vectors; and the recombinant viral vector is selected from poxviruses (e.g., Tian Tan strain, North American vaccine strain, Wyeth derivative strain, Listeria strain, Ankara derivative strain, Copenhagen strain, and New York strain), adenoviruses (e.g., adenovirus types 5, 11, 26, 35, 63, and 68), adeno-associated viruses, herpes simplex virus, measles virus, enterovirus, reovirus, rhabdovirus, flavivirus, influenza virus, parainfluenza virus, respiratory syncytial virus, and poliovirus vectors.

Expression vectors comprising immunogenic peptide encoding sequences and suitable transcription/translation control signals can be constructed using conventional methods in the art. These methods include *in vitro* recombinant DNA technology, DNA synthesis technology, *in vivo* recombination technology, etc. The DNA sequence can be effectively/operably linked to an appropriate promoter in the expression vector to guide mRNA synthesis. The expression vector also comprises a transcription terminator and a ribosome binding site for translation initiation. Expression systems such as pcDNA3.1 vector, pIRES2-EGFP vector, AdMax^{™}, and AdC68 can be used in the present disclosure. Furthermore, the expression vector may comprise one or more selective marker genes to provide phenotypic traits for selecting transformed host cells, such as dihydrofolate reductase, neomycin resistance, and green fluorescent protein (GFP) for eukaryotic cell culture, or tetracycline or ampicillin resistance for *Escherichia coli* (*E. coli*).

The vector containing the above-mentioned appropriate DNA sequence and an appropriate promoter or control sequence can be used to transform appropriate host cells to enable them to express proteins or polypeptides. The host cell can be a prokaryotic cell, such as a bacterial cell; or a lower eukaryotic cell, such as a yeast cell; or a higher eukaryotic cell, such as an animal cell. Representative examples include *Escherichia coli,* Streptomyces genus, Agrobacterium genus; fungal cells such as yeast; and animal cells. In the present disclosure, host cells selected from, for example, the group consisting of HEK293, HeLa, CHO, K562, NS0, SP2/0, PER.C6, Vero, RD, BHK, HT 1080, A549, Cos-7, ARPE-19 and MRC-5 cells; High Five, Sf9, Se301, SeIZD2109, SeUCR1, Sf9, Sf900+, Sf21, BTI-TN-5B1-4, MG-1, Tn368, HzAm1, BM-N, Ha2302, Hz2E5 and Ao38, may be used.

If necessary, the recombinant protein can be separated and purified by various separation methods based on its physical, chemical and other properties. These methods are well known to those skilled in the art. Examples of these methods include, but are not limited to conventional refolding treatment, treatment with protein precipitants (salting out), centrifugation, permeabilization, sonication, ultracentrifugation, molecular sieve chromatography (gel filtration), adsorption chromatography, ion exchange chromatography, high-performance liquid chromatography (HPLC), and various other liquid chromatography techniques and combinations thereof.

### Vaccines and Immunoconjugates

The present disclosure also provides a vaccine, or immune composition, comprising the immunogenic peptides, nucleic acid molecules, vectors, and/or host cells of the present disclosure. The vaccine comprises a formulation of the immunogenic peptides and/or nucleic acid molecules of the present disclosure, in a form capable of being administered to vertebrates (preferably mammals), and it induces a protective immune response that enhances immunity to prevent and/or mitigate influenza A virus infection.

The term "protective immune response" or "protective response" refers to an immunogen-mediated immune response against an infectious agent or disease, as exhibited in vertebrates (e.g., humans), that prevents or reduces infection with influenza A or reduces at least one of the symptoms thereof.

The terms "vertebrate" or "subject" or "patient" refer to any member of the subphylum Chordata, including, but not limited to humans and other primates, including non-human primates such as chimpanzees and other apes and monkeys; livestock such as cattle, sheep, swine, goats, and horses; domesticated mammals such as dogs and cats; laboratory animals, including rodents such as mice, rats, and guinea pigs; and birds including domesticated, wild, and game birds such as chickens, turkeys, and other quail birds, ducks, and geese. The terms "mammal" and "animal" are encompassed within the definition to cover adult, juvenile, and newborn individuals.

The vaccines described herein may be mRNA vaccines, recombinant protein vaccines, recombinant DNA vaccines, recombinant viral vector vaccines (e.g., adenovirus vector, poxvirus vector, adeno-associated virus vector, herpes simplex virus vector, cytomegalovirus vector), recombinant bacterial vector vaccines, recombinant yeast vector vaccines, or recombinant virus-like particle vaccines. In some embodiments, the vaccines described herein are preferably mRNA vaccines.

The vaccine compositions described herein comprise an effective amount of the immunogen described herein or an encoding molecule thereof. The term "effective amount" generally refers to the amount of the immunogen or an encoding molecule thereof that can induce a protective immune response sufficient to induce immunity to prevent and/or mitigate infection or disease and/or reduce influenza A infection or at least one of the symptoms thereof.

The vaccines described herein may also comprise adjuvants. Adjuvants known to those skilled in the art may be used, such as those described in Vogel et al., A Compendium of Vaccine Adjuvants and Excipients (2nd edition) (which are incorporated herein by reference in its entirety). Examples of known adjuvants include, but are not limited to aluminum adjuvants, cholera toxin and subunits thereof, oligodeoxynucleotides, manganese ion adjuvants, colloidal manganese adjuvants, Freund's adjuvant, MF59 adjuvant, QS-21 adjuvant, Poly I:C and other TLR ligands, GM-CSF, IL-2, IL-3, IL-7, IL-11, IL-12, IL-18, IL-21, etc.

The vaccine compositions described herein may also include pharmaceutically acceptable carriers, diluents, preservatives, solubilizers, emulsifiers, and other excipients. For example, pharmaceutically acceptable carriers are known and include, but are not limited to, water for injection, saline solutions, buffered saline, dextrose, water, glycerol, sterile isotonic buffers, and combinations thereof. Pharmaceutically acceptable carriers, diluents, and other excipients can be found, for example, in Remington 's Pharmaceutical Sciences.

In some embodiments, the nucleic acid expression vector (e.g., mRNA) is contained alone in the packaging material or in combination with a carrier in a delivery system. For example, the delivery system is selected from lipid delivery systems, lipid-like delivery systems, polymer delivery systems, or combinations thereof, such as loaded on lipid nanoparticles, cationic liposomes, polyurethane (PAA), poly- β-amino ester (PBAE), polyethyleneimine (PEI), or lipid-encapsulated polymer micelles. In some embodiments, the nucleic acid expression vector is combined with liposome nanoparticles. In some embodiments, the liposome nanoparticles comprise a combination of cationic lipids, structural lipids, accessory lipids, and stabilizing lipids.

In some embodiments, the liposome nanoparticles, calculated by molar percentage, comprise 20-50% cationic lipids: 20-50% structural lipids: 5-20% accessory lipids: 1-5% stable lipids, more preferably 50% cationic lipids: 38% structural lipids: 10% accessory lipids: 2% stable lipids.

In some embodiments, the cationic lipids in the liposome nanoparticles are one or more selected from the group consisting of (2,3-dioleoyl-propyl)-trimethylammonium chloride (DOTAP), didecyl adipate (DDA), 3β-[N-(N',N'-dimethylaminoethyl)carbamoyl]cholesterol (DC-Chol), N,N-dimethyl-4-pyridinamine (DMAP), 1,2-triethanolamine-3-trimethylpropane (DOTMA), N-[1-(2,3-dioleoyl)propyl]-N-(arginine-aminoamide)ethyl-N,N-dimethyltrifluoroacetate ammonium (DOSPA), methyl (dilinoleyl) 4-(N,N-dimethylamino)butanoate (Dlin-MC3-DMA), KC2, N,N-dimethyl-2,2-di-(9Z, 12Z)-9,12-octadecadien-1-yl-1,3-dioxolane-4-ethylamine (Dlin-KC2-DMA), preferably methyl (dilinoleyl) 4-(N,N-dimethylamino)butanoate (Dlin-MC3-DMA).

In some embodiments, the structural lipids in the liposome nanoparticles include cholesterol, cholesterol esters, steroid hormones, steroid vitamins, or bile acids, preferably cholesterol.

In some embodiments, the accessory lipids in the liposome nanoparticles include dipalmitoylphosphatidylcholine (DPPC), dioleoylphosphatidylglycerol (DOPG), 1-palmitoyl-2-oleoyllecithin (POPC), 1,2-distearoyl-3-phosphatidylethanolamine (DSPE), dioleoyllecithin (DOPC), dioleoylphosphatidylserine (DOPS), distearate phosphatidylcholine (DSPC), preferably distearate phosphatidylcholine (DSPC).

In some embodiments, the stabilizing lipids in the liposome nanoparticles include polyethylene glycol (PEG) lipids, such as PEG-modified phosphatidylethanolamine, PEG-modified phosphatidic acid, PEG-modified ceramide, and PEG-modified diacylglycerol, preferably PEG-modified diacylglycerol, and more preferably 1,2-dimyristoyl-rac-glycerol-3- methoxy polyethylene glycol 2000 (PEG 2000-DMG) for long-circulating liposomes.

In some embodiments, the mass ratio of cationic liposomes to mRNA in the liposome nanoparticles can be 10-30:1, preferably 15-20:1.

In some embodiments, cationic lipid nanoparticles are prepared by mixing cationic liposomes Dlin-MC3-DMA, cholesterol, phospholipid DSPC, and PEGylated lipid DMG-PEG2000 in anhydrous ethanol to obtain a mixed organic phase, which is then mixed with a suitable buffer system (e.g., citrate buffer) to obtain a lipid mixture. The lipid mixture is then extruded and filtered, for example, using an extruder, to obtain cationic liposome nanoparticles. The prepared mRNA is dissolved in a suitable buffer system (e.g., citrate buffer), added to the cationic lipid nanoparticle mixture, thoroughly mixed, and incubated. The mixture is then dialyzed (e.g., overnight with sterile PBS) to obtain cationic lipid nanoparticles encapsulating the mRNA. In some embodiments, the ratio of the organic phase to the aqueous phase is 1:2 to 1:5, preferably 3:7.

The vaccine compositions described herein are available in forms suitable for systemic or local (especially intrarespiratory) administration. Methods of administration of the vaccine compositions include, but are not limited to intramuscular injection, intradermal injection, subcutaneous injection, intranasal drops, nebulized inhalation, genitourinary administration, rectal administration, oral administration, or any combination thereof, such as intramuscular injection followed by intranasal drops.

In some embodiments, the vaccine described herein prevents, eliminates, or reduces influenza A virus and at least one of symptoms thereof in the subject, such as respiratory symptoms (e.g., nasal congestion, sore throat, hoarseness), headache, cough, sputum, fever, rales, wheezing, difficulty breathing, pneumonia due to infection, severe acute respiratory syndrome, renal failure, etc.

The present disclosure also relates to an immunoconjugate (also known as an immune conjugate) comprising the immunogen described herein and other substances conjugated thereto. These other substances may be targeting substances (such as portions that specifically recognize a particular target), therapeutic substances (such as drugs, toxins, or cytotoxic agents), or labeling substances (such as fluorescent labels or radioisotope labels).

The present disclosure also provides a combination product comprising the immunogenic peptides, nucleotide molecules, vectors, host cells, and/or vaccines of the present disclosure, and may further comprise one or more other substances that contribute to better prevention and/or treatment of influenza virus infection or symptoms thereof, or enhance the stability of the aforementioned substances. For example, other substances may comprise other vaccines against influenza viruses, such as other vaccines against influenza virus HA or HA2, said HA or HA2 being derived from, but not limited to H1-H18; and other active substances for diseases or conditions that benefit from T cell activation and/or memory immune responses to T cells, such as T cell antigens.

### Immunization method(s)

The present disclosure also provides a method for preventing and/or treating influenza A virus infection and/or its symptoms, comprising administering at least one dose of a preventive and/or therapeutically effective amount of one or more vaccines of the present disclosure. Possible routes of administration include, but are not limited to systemic immunization, such as intramuscular, subcutaneous, and intradermal injections; and intrarespiratory immunization, such as nebulization or intranasal drops. In some embodiments, primary immunization is performed using systemic or intrarespiratory administration, with systemic administration being preferred.

In some embodiments of the present disclosure, the interval between two vaccinations is at least one week, such as two weeks, four weeks, two months, three months, six months or longer.

In some embodiments, the immunization method of the present disclosure may adopt a "prime-boost" or "prime-boost-reboost" approach, and may employ a single systemic immunization or a respiratory local immunization approach, or a combination of two immunization approaches.

The combination products described herein may be provided in the form of drug packages or kits, for example, one or more vaccine compositions or one or more components thereof may be packaged in one or more containers, such as sealed containers, such as ampoules or capsules, specifying the amount of the composition. Vaccine compositions may be provided in the form of liquids, sterile lyophilized powders, or anhydrous concentrates, and may be diluted, reconstituted, and/or formulated with appropriate liquids (e.g., water, saline, etc.) to obtain the appropriate concentration and form for administration to the recipient prior to use.

### Examples

To further illustrate the technical means and effects of the invention, the following detailed description is provided in conjunction with specific examples. It is to be understood that the specific examples described herein are merely illustrative of the invention and not intended to limit its scope. Those skilled in the art can make appropriate modifications and variations to the embodiments described herein, all of which are within the scope of the present disclosure.

For experimental methods where specific techniques and/or conditions are not indicated in the following examples, conventional methods well known in the art may be used, such as those referenced in "Molecular Cloning: A Laboratory Manual" (3rd edition, Cold Spring Harbor Laboratory Press, New York, 1989) or according to conditions recommended by suppliers. All reagents and instruments used are commercially available products. Unless otherwise stated, the test methods may be conventional methods in the art or may be provided by commercial companies.

Unless otherwise stated, percentages and parts are calculated by weight. Unless otherwise defined, all technical and scientific terms used herein have the same meanings as commonly understood by those skilled in the art. Furthermore, any methods and materials similar or equivalent to those described herein may be used in the methods of the present disclosure. The preferred implementation methods and materials described herein are for illustrative purposes only.

### Materials, Methods and Animals

The mRNA preparation, cationic liposome nanoparticle preparation, animal immunization protocol, and detection methods involved in the experiments described in the examples are summarized below.

### I . mRNA preparation

A DNA sequence containing the target gene was used as the basic building unit. After optimization based on eukaryotic codon bias, it served as the template for subsequent *in vitro* transcription experiments (all sequences containing the HA2 fragment in the examples contain N82R and N154H mutations) (the sequences used can be found in the appendix of the specification and the attached sequence listing). The method for obtaining the basic sequence of the T-cell antigen is as described in the Chinese patent application 201880001963.X, and the sequence was optimized according to the specific needs of the present application. The amino acid and nucleotide sequences of the T-cell antigen used in the examples can be found in SEQ ID NOs: 11 and 12.

The basic process of *in vitro* transcription is as follows: a plasmid containing the target gene constructed in the laboratory is subjected to single-enzyme digestion using XbaI (Thermo Fisher Scientific, FD0685) and then recovered and purified as a template; transcription reagents such as NTPs are added; the transcription is performed using T7 polymerase and purified to obtain the corresponding mRNA; then, the capping step is completed via enzymatic capping, and the mRNA is purified with lithium chloride and stored at -80°C for later use.

### II . Preparation of cationic lipid nanoparticles (LNPs) for encapsulating mRNA

To prepare cationic lipid nanoparticles, cationic lipid Dlin-MC3-DMA, cholesterol, phospholipid DSPC, and PEGylated lipid DMG-PEG2000 were mixed in anhydrous ethanol at a molar ratio of 50:38:10:2 to obtain a mixed organic phase; and the phase was then mixed with 50 mM citrate buffer (pH 4.0) at a mass ratio of 20:1 to obtain a lipid mixture. This lipid mixture was then added to an extruder for extrusion and filtration to obtain cationic liposome nanoparticles. The prepared mRNA was dissolved in citrate buffer and added to the cationic lipid nanoparticle mixture at a volume ratio of 1:1. After thorough mixing, the mixture was incubated at 42 °C for 1 hour, followed by dialysis overnight against sterile PBS. The resulting mixture was then sterile filtered through a 0.22 µm filter to obtain cationic lipid nanoparticles encapsulating the mRNA.

### III. Animal Immunization Protocol

FemaleBALB/c mice aged 6-8 weeks at the time of primary immunization were selected, with 14 mice in each experimental group and control group (for challenge with two subtypes of influenza virus, the mice were further divided into two subgroups, with 7 mice for each virus). All groups were immunized intramuscularly. The primary immunization was performed on day 0, and booster immunizations were performed on day 28 (i.e., 4 weeks). The dosage for each immunization was the same: 5 µg of the corresponding mRNA per mouse, and the volume of each immunization was 50 µL.

### IV. Animal challenge protocol

Three weeks after booster immunization, mice in each immunized group were randomly and equally divided into two subgroups.

*Subgroup 1*: For the challenge with 1000 TCID₅₀ of pdmH1N1 virus (i.e., A/California/04/2009) intranasally, mice were first anesthetized by intraperitoneal injection of 2.5% tert-amyl alcohol and 1.25% tribromoethanol at a dose of 15 mL/kg body weight, and once deeply anesthetized, a total of 25 µL of diluted virus solution was instilled into both nostrils. Infection symptoms were observed after challenge, and body weight changes and survival rates were recorded over 14 days.

*Subgroup 2:* Mice were challenged with 500 TCID₅₀ of H3N2 using the same method as subgroup 1. The infection symptoms of mice after challenge were observed and the weight changes and survival rate were recorded over 14 days.

### V. Detection Methods

### Blood collection

Blood samples were collected from mice via the retro-orbital venous plexus at weeks 4, 5, and 6 after the primary immunization, and the mice, under anesthesia, were challenged three weeks after the final immunization. The collected whole blood from the mice was placed in 1.5 mL sterile EP tubes, incubated at 37 °C for 30 minutes to accelerate coagulation, and then centrifuged at 7000 × g for 15 minutes to separate serum. The serum was then inactivated at 56 °C for 30 minutes to inactivate complements.

### Serum IgG antibody titer detection using enzyme-linked immunosorbent assay (ELISA)

The HA protein of the H7N9 strain, or the M2e protein, was diluted with coating buffer (50 mM carbonate buffer, pH 9.6) to a final concentration of 1 µg/mL. 100 µL of diluted antigen was added to each well of a 96-well ELISA plate and incubated overnight at 4 °C to coat the ELISA plate. After coating, PBST containing 5% skim milk was used as a blocking buffer, and the plate was incubated at 37 °C for 2 h to block sites on the ELISA plate where no protein had been adsorbed. After blocking, mouse serum samples were added to the first well of the blocked ELISA plate for a 1:100 initial dilution, followed by two-fold serial dilutions for antibody incubation. After incubation at room temperature for 3 h, the unbound antibodies were washed away, followed by incubation with a secondary antibody (HRP-labeled goat anti-mouse IgG antibody (1:5000)). After the incubation, the unbound secondary antibody was washed away and color development was initiated. After color development, the reaction was terminated and the plate was read using a microplate reader. The ELISA endpoint method was employed, absorbance was measured at 490 nm, and the plate reading results were recorded.

### Detection of T cells that specifically recognize M2e or HA2 and secrete IFN-γ using enzyme-linked immunospot (ELISpot) assay.

First, a plate was taken out and the coating antibody was diluted with sterile PBS (1:200) and added to the plate at 100 µL/well, then incubated overnight at 4 °C for coating. After being washed once with RPMI 1640 medium containing 10% fetal bovine serum (FBS), the plate was blocked with the same medium at room temperature for 2 h. After removing the medium, stimuli were added to the wells, followed by lymphocytes from mouse spleens (2.0 × 10⁵ cells/well; alternatively, the stimuli may be pre-mixed with the cells before being added to the plate). In this experiment, the stimuli were an M2e peptide pool or an HA2 peptide pool (Qiangyao Biotechnology), diluted to a final concentration of 5 µg/mL. Duplicate wells were prepared for each mouse. Additionally, background controls and blank controls were set up on the plate. The background controls were wells containing only cell-free medium, to exclude non-specific color development caused by the medium; the blank controls were wells containing cells but without stimuli, to exclude IFN-γ secretion by cells due to external non-experimental factors. The plate was incubated at 37 °C in a 5% CO₂ incubator for 12-48 h. The plate was then emptied, washed twice with pre-cooled ddH2004, and left to stand for 3 minutes each time, followed by washing three times with PBST (200 µL/well). The detection antibody was diluted with PBS containing 10% FBS (PBS-10% FBS), and then added to the plate (1:250, 100 µL/well). After incubation at room temperature for 2 h, the plate was washed. Streptavidin-HRP diluted with PBS-10% FBS (1:100, 100 µL/well) was added, and incubated at room temperature for 1 h, followed by washing. A color-developing solution (100 µL/well) was added. When clearly visible spots appeared, the reaction was terminated by washing the plate. The plate was dried and read, and the results were statistically analyzed.

### Example 1. Verification of expression of mRNA transfected into HEK293T cells

The prepared mRNA was transfected into HEK293T cells to verify expression. On day 0, HEK293 cells were seeded into a 6-well plate at a density of 800,000 cells per well using DMEM complete medium containing 10% fetal bovine serum. On day 1, transfection was performed using transfection reagent, Lipofectamine 3000, with 3 µg mRNA per well. After incubation at 37 °C for 45-48 h, the cells were taken out. Cells were collected by pipetting and lysed on ice for 1 h with 100 µL RIPA lysis buffer, followed by centrifugation at 12,000 g for 10 min at 4 °C, and the supernatant was collected. Appropriate loading buffer was added and samples were subjected to SDS-polyacrylamide gel electrophoresis (SDS-PAGE), followed by membrane transfer. The membrane was blocked with 5% skim milk at room temperature for 2 h and incubated overnight at 4 °C with a primary antibody (mouse anti-M2e monoclonal antibody, Thermo Fisher, Cat. MA1-082, 1:3000). After washing the membrane with PBST (PBS containing 0.05% Tween-20), it was incubated with a goat anti-mouse secondary antibody (Yeasen Biotechnology, Cat. 33201ES60, 1:5000) for 1 h at room temperature, followed by washing with PBST (containing 0.05% Tween-20). Chemiluminescent detection was performed and images were captured.

The results are shown in Fig. 1B. The eight tandem repeats of M2e (8M2e), 8M2e-HA (M8H), and 8M2e-HA-collagen (M8HC) were all clearly expressed and recognized by specific antibodies.

### Example 2. Humoral immune responses induced in mice by mRNA vaccines 8M2e, M8H, and M8HC

The normal expression of the mRNAs (8M2e, M8H, and M8HC) in this example was verified in Example 1. Cationic lipid nanoparticles encapsulating each of these three mRNAs were prepared using Experimental Method II.

BALB/c mice were immunized with lipid nanoparticle vaccines encapsulating each of these three mRNAs to evaluate immunogenicity, respectively. Each group contained twelve mice, and 50 µL of liposomes encapsulating 5 µg of the corresponding mRNA were intramuscularly injected into hind limbs of each mouse. The negative control was an equal volume of cationic lipid nanoparticles without encapsulating nucleic acids (empty liposomes). As shown in Fig. 2A, the immunization schedule was two immunizations at a 4-week interval. After primary immunization with three different mRNA vaccines and the empty liposomes for control groups at week 0, a booster immunization at week 4 was conducted. Peripheral blood was collected from mice at weeks 4, 5, and 6 and sera were isolated. Enzyme-linked immunosorbent assay (ELISA) was used to detect the titers of antibodies specifically binding to HA and M2e proteins.

As shown in Figs. 2B and 2C, at week 4 after the primary immunization, the geometric mean titers (GMTs) of M2e antibodies in sera of immunized mice from 8M2e, M8H, and M8HC groups were 1076, 328, and 328, respectively, while the GMTs of HA antibodies in sera of immunized mice from M8H and M8HC groups were 164 and 148, respectively. A booster immunization was performed after blood collection at week 4. At week 5 (one week after the booster), the GMTs of M2e antibodies in sera of immunized mice from 8M2e, M8H, and M8HC groups were 76082, 21000, and 102400, respectively, while the GMTs of HA antibodies in sera of immunized mice from M8H and M8HC groups were 148 and 362, respectively. At week 6 (two weeks after the booster), the GMTs of M2e antibodies in sera of immunized mice from 8M2e, M8H, and M8HC groups were 92746, 25600, and 204800, respectively. The GMTs of HA antibodies in sera of immunized mice from M8H and M8HC groups were 297 and 362, respectively.

At week 7 (three weeks after booster), mice were anesthetized and challenged intranasally. Mice in each immunized group were randomly and equally divided into two subgroups, one challenged with H1N1 and the other with H3N2. The day of challenge was designated as day 0, and body weight and survival were monitored for 14 consecutive days.

Figs. 2D and 2F show results of body weight and survival following HIN1 challenge.
Body weight decreased in all groups after challenge. Except for the empty control groups, the body weight of mice in other groups began to recover after day 7 or day 8. All mice in the M8HC group survived, whereas survival rates were 83% in the M8H group and 67% in the 8M2e group. Figs. 2E and 2G show results of body weight and survival following H3N2 challenge. All groups of mice experienced a significant decrease in body weight after challenge. Survival rates showed that 50% of the M8HC group survived, 17% of the M8H group survived, while all mice in the 8M2e group died. All mice in the control groups died.

These results show that after the primary immunization, the binding antibody titers against M2e and HA proteins in the serum of each group were only 100 to 1000. However, after the booster immunization, the serum antibody titers induced in each group were significantly increased. The binding antibody titers against M2e increased to 100,00-100,000 as early as one week after the booster, and the antibody titers further increased over time. Following H1N1 challenge, all groups experienced weight loss, but only the M8HC group showed 100% survival, while the 8M2e group had 67% survival, indicating that antibodies against M2e alone could not provide full protection. Following H3N2 subtype challenge, 50% of the M8HC group mice survived, and 17% of the M8H group mice survived.

These results demonstrate that mRNAs formed by linking M2e and HA2 can effectively induce immune responses against both M2e and HA2, thereby providing excellent protection against different subtypes of influenza A virus infection. Furthermore, the inventors unexpectedly discovered that further linking a low-immunogenic multimerized motif C (collagen) to the M2e and HA fusion could more effectively induce an immune response and achieve even better protective effects.

### Example 3. Humoral immune responses induced in mice by mRNA vaccines M8HC and M4HC

Fig. 3A illustrates the constructions of M8HC and M4HC. Normal expressions of the mRNA (M8HC) in this example were verified in Example 1. mRNA (M4HC) was transfected and its expression was verified using the method in Example 1, as shown in Fig. 3B, M4HC was also expressed normally. Cationic lipid nanoparticles encapsulating each of these two mRNAs were prepared using Experimental Method II.

BALB/c mice were immunized with lipid nanoparticle vaccines containing these two mRNAs to evaluate their immunogenicity. Each group contained fourteen mice. 50 µL of liposomes encapsulating 5 µg of the corresponding mRNA were intramuscularly injected into hind limbs of each mouse. The negative controls were empty liposomes. The immunization procedure was the same as in Example 2. Peripheral blood was collected from mice at weeks 4, 5, and 6 and sera were isolated. Enzyme-linked immunosorbent assay (ELISA) was used to detect the titers of antibodies specifically binding to HA and M2e proteins.

As shown in Figs. 3C and 3D, at week 4 after the primary immunization, the GMTs of M2e antibodies in sera of immunized mice from M8HC and M4HC groups were 328 and 121, respectively; while the GMTs of HA antibodies in sera of immunized mice from M8HC and M4HC groups were 148 and 134, respectively. Booster immunizations were performed after blood collection at week 4. At week 5 (one week post-boost), M2e antibody GMTs were 102,400 and 1,766 for M8HC and M4HC immunized mice groups, respectively, while HA antibody GMTs were 362 and 975. At week 6 (2 weeks post-boost), M2e antibody GMTs were 204,800 and 8,613, while HA antibody GMTs were 362 and 883, respectively.

At week 7 (three weeks after booster), mice were anesthetized and challenged intranasally. Mice in each immunized group were randomly and equally divided into two subgroups, one challenged with H1N1 and the other with H3N2. The day of challenge was designated as day 0, and body weight and survival were monitored for 14 consecutive days.

Figs. 3E and 3G show the results of weight and survival following HIN1 challenge. Mice in both M8HC and M4HC groups experienced gradual body weight loss after the challenge, reaching the lowest average body weight on day 7, followed by a gradual recovery. In contrast, body weight in the empty liposome control groups declined rapidly until death. Survival results showed that all mice in M8HC and M4HC groups survived, while all mice in the control group died. Figs. 3F and 3H show the results of weight and survival following H3N2 challenge. All groups of mice experienced a rapid decrease in body weight after challenge, but the average body weight of mice in the M4HC group decreased significantly more slowly than that of the M8HC group. Survival results showed that 50% of mice in the M8HC group survived, while 83% of mice in the M4HC group survived, and all mice in the control group died.

These results indicate that after reducing the number of M2e repeats in the immunogen from 8 to 4, the binding antibody titers against M2e and HA proteins in sera of each group were only 100-1000 after the primary immunization, which was not significantly different from the results in Example 2. However, after the booster immunization, the binding antibody titer against M2e in the serum of the M4HC group was significantly lower than that of the M8HC group, whereas the average binding antibody titer against HA increased by approximately 2.7-fold. These results suggest that, after the number of M2e repeats was reduced, the antibodies induced against M2e were decreased while the antibodies against HA were increased, thereby resulting in a more balanced antibody response to the two immunogens. Following H1N1 challenge, all mice in M8HC and M4HC groups survived, suggesting that reduction of M2e repeats did not compromise protective efficacy. The H3N2 challenge results suggested that M4HC conferred better protection against H3N2 subtype compared to M8HC.

The above results demonstrate that mRNAs formed by linking M2e, HA2, and multimerized motifs can effectively induce immune responses against both M2e and HA2, thereby providing excellent protection against different subtypes of influenza A virus infection. Furthermore, by adjusting the number of M2e repeats, the balance of responses to the two immunogens, M2e and HA, can be modulated, enhancing protection against more viral subtypes and broadening the protective spectrum.

### Example 4. Humoral and cellular immune responses induced in mice by mRNA vaccines M4HC and M4HF

Fig. 4A illustrates the constructions of M4HC and M4HF. In this example, the newly constructed mRNA (M4HF) was transfected and its expression was verified using the method described in Example 1. As shown in Fig. 4B, M4HF was expressed normally. Cationic lipid nanoparticles encapsulating M4HC or M4HF were prepared using Experimental Method II, respectively.

BALB/c mice were immunized with lipid nanoparticle vaccines containing these two mRNAs to evaluate their immunogenicity. Each group contained fourteen mice. 50 µL of liposomes encapsulating 5 µg of the corresponding mRNA were intramuscularly injected into hind limbs of each mouse. The negative controls were empty liposomes. The immunization procedure was the same as in Example 2. Peripheral blood was collected from mice at weeks 4, 5, and 6 and sera were isolated. ELISA was used to detect the titers of antibodies specifically binding to HA and M2e proteins.

As shown in Figs. 4C and 4D, at week 4 after the primary immunization, the GMTs of M2e antibodies in sera of immunized mice from M4HC and M4HF groups were 121 and 121, respectively; while the GMTs of HA antibodies in sera of immunized mice from M4HC and M4HF groups were 134 and 297, respectively. Booster immunizations were performed after blood collection at week 4. At week 5 (one-week post boost), M2e antibody GMTs were 1,766 and 56,529 for M4HC and M4HF immunized mice groups, respectively, while HA antibody GMTs were 1,188 and 2,377. At week 6 (2 weeks post-boost), M2e antibody GMTs were 8,613 and 11,593, while HA antibody GMTs were 883 and 3,533, respectively.

At week 7 (three weeks after booster), mice were anesthetized and challenged intranasally. Mice in each immunized group were randomly and equally divided into two subgroups, one challenged with H1N1 and the other with H3N2. The day of challenge was designated as day 0, and body weight and survival were monitored for 14 consecutive days.

Figs. 4E and 4G show the results of weight and survival following H1N1 challenge. Mice in both M4HC and M4HF groups experienced gradual body weight loss after the challenge, but the weight gradually recovered after day 7. Survival results showed that all mice in M4HC and M4HF groups survived, while all mice in the control group died. Figs. 4F and 4H show the results of weight and survival following H3N2 challenge. All groups of mice experienced a rapid decrease in body weight after challenge, with an average decrease of more than 10%. Body weight gradually recovered starting on day 8. Survival results showed that all mice in the M4HF group survived, while 83% of the mice in the M4HC group survived, and all mice in the control group died.

The reactivity of T lymphocytes to immunogens after vaccine immunization was detected, and the results are shown in Figs. 4I and 4J. 10 days after booster immunization, mice in both the M4HC and M4HF groups produced T cells that could specifically recognize M2e and HA2. Among them, the number of T cells responding to the M2e peptide pool was approximately 1100 per 10⁶ cells (M4HC) or approximately 1900 per 10⁶ cells (M4HF), and the number of T cells responding to the HA2 peptide pool was approximately 58 per 10⁶ cells (M4HC) or approximately 105 per 10⁶ cells (M4HF), both of which were significantly higher than those in the liposome empty control groups.

The experimental results show that using different multimer sequences can alter or improve the immunogenicity of composite immunogens. In this example, collagen was replaced by Foldon to further enhance the immunogenicity of immunogens. Specifically, after booster immunization, the binding antibody titers against M2e and HA in mice sera of the M4HF group were higher than those in the M4HC group. One week after booster, the binding antibody titer against M2e in mice sera of the M4HF group was 32 times that of the M4HC group, and the antibody titer against HA was twice that of the M4HC group. The number of T cells specifically recognizing M2e and HA2 in mice was also significantly higher than that in the control group. After H1N1 challenge, all mice in both the M4HC and M4HF groups survived. After H3N2 challenge, only mice in the M4HF group all survived. These results suggest that the M4HF group not only significantly improved antibody titers compared to the M4HC group, but also provided better protection against more subtypes with the vaccine.

The above results demonstrate that mRNAs formed by linking M2e, HA2, and multimerized motifs can effectively induce immune responses against both M2e and HA2, thereby providing excellent protection against various influenza virus infections. Furthermore, by adjusting the polymerizing motifs used, the level of the immune response can be altered or improved (even significantly improved), providing broad-spectrum and highly effective protection.

### Example 5. Humoral and cellular immune responses induced in mice by mRNA vaccines M4HF-T and M4HF+T

Fig. 5A illustrates the constructions of M4HF-T and T. In this example, the newly constructed mRNA (M4HF-T, T) was transfected and its expression was verified using the method described in Example 1. The M4HF-T comprised an IRES- linked M4HF (SEQ ID NO : 27) peptide segment and a T-cell antigen (SEQ ID NO : 11) peptide segment linked to DHFR (SEQ ID NO : 43). Results showed that both M4HF-T and the T-cell antigen peptide could be expressed normally. Experimental method II was used to separately prepare (a) mRNA cationic liposome nanoparticles encapsulating M4HF-T, or (b) a mixture of M4HF+T mRNA cationic liposome nanoparticles, wherein M4HF and T were separately encapsulated in different liposome particles, and then the two types of nanoparticles were mixed at an mRNA mass ratio of 1:3.

BALB/c mice were immunized with lipid nanoparticle vaccines encapsulating either ① M4HF-T or ② M4HF+T mRNA, respectively, to evaluate their immunogenicity. Each group contained fourteen mice. 150 µL of liposomes encapsulating 20 µg of the corresponding mRNA (M4HF:T = 1:3) were intramuscularly injected into both hind limbs of each mouse. The negative controls were empty liposomes. The immunization procedure was the same as in Example 2. Peripheral blood was collected from mice at weeks 5 and 6 and serawere isolated. ELISA was used to detect the titers of antibodies specifically binding to HA and M2e proteins.

At week 7 (three weeks after booster), mice were anesthetized and challenged intranasally. Mice in each immunized group were randomly and equally divided into two subgroups, one challenged with H1N1 and the other with H3N2. The day of challenge was designated as day 0, and body weight and survival were monitored for 14 consecutive days.

Results of body weight and survival following H1N1 challenge showed that mice in the M4HF-T and M4HF + T groups gradually lost body weight after challenge, and survival results showed that mice in both groups had high survival rates, while all mice in the control group died. Results of body weight and survival following H3N2 challenge showed that mice in the M4HF-T and M4HF +T groups experienced a decrease in average body weight after challenge, followed by a gradual recovery, and survival results showed that mice in both groups had high survival rates, while all mice in the control group died.

At week 6 (two weeks after booster immunization), five mice from each group were sacrificed, their spleens were removed, lymphocytes were isolated, and the number of specific T cells secreting IFN-γ was detected using ELISpot. The results showed that the immunized mice in the M4HF-T and M4HF+T groups produced a large number of specific T cells, which were significantly higher than those in the control mice.

The experimental results showed that adding T-cell antigens to the composite immunogen resulted in good immune responses in mice. Specifically, mice exhibited high survival rates after H1N1 or H3N2 challenge and produced a large number of specific T cells. These results suggest that adding the immunogen component improved the protective efficacy of the vaccine.

### Example 6. Protection of mice by mRNA vaccines M4H1F, M4H3F, M2HF, M4H and HF

Fig. 6A illustrates the constructions of M4H1F, M4H3F, M2HF, M4H and HF. The amino acid sequences of M4H1F, M4H3F, M2HF, M4H, and HF are set forth in SEQ ID NO: 29, SEQ ID NO: 31, SEQ ID NO: 33, SEQ ID NO: 35, and SEQ ID NO: 37, respectively, or are encoded by nucleotide molecules having the sequences set forth in SEQ ID NO: 30, SEQ ID NO: 32, SEQ ID NO: 34, SEQ ID NO: 36, and SEQ ID NO: 38, respectively. In this example, the newly constructed mRNAs (M4H1F, M4H3F, M2HF, M4H, and HF) were transfected and their expression was verified using the method described in Example 1. The results showed that M4H1F, M4H3F, M2HF, M4H, and HF could all be expressed normally. Cationic lipid nanoparticles encapsulating above five types of mRNAs were prepared using Experimental Method II, respectively.

BALB/c mice were immunized with lipid nanoparticle vaccines encapsulating M4H1F, M4H3F, M2HF, M4H and HF, respectively, to evaluate their immunogenicity. Each group contained fourteen mice. 50 µL of liposomes encapsulating 5 µg of the corresponding mRNA were intramuscularly injected into hind limbs of each mouse. The negative controls were empty liposomes. The immunization procedure was the same as in Example 2.

At week 7 (three weeks after booster), mice in M4H1F, M4H3F, M2HF, M4H and HF groups were anesthetized and challenged intranasally. Mice in each immunized group were randomly and equally divided into two subgroups, one challenged with H1N1 and the other with H3N2. The day of challenge was designated as day 0, and body weight and survival were monitored for 14 consecutive days.

Experimental results showed that reducing the number of M2e repeats to two significantly decreased the induced M2e antibody titer. The protective effects of HF containing only HA2 and the multimer, and MH containing both M2e and HA2 but without the multimer, were significantly worse than those of M4HF. Replacing the HA2 portion of the vaccine with the previously reported H1N1 or H3N2 source resulted in a decrease in the level of broad-spectrum protective antibodies. These results demonstrate that composite immunogens containing the HA2 portion derived from H7N9 can induce a broader-spectrum immune response.

All references cited herein are incorporated by reference in their entirety as though each reference was individually incorporated by reference. In addition, it should be understood that after reading the contents of the present disclosure, those skilled in the art may make various changes or modifications to the present disclosure, and these equivalent forms also fall within the scope defined by the appended claims of the present application.

### Appendix: Sequence information

| **Amino acid sequences (SEQ ID NO)** | **Nucleotide sequences (SEQ ID NO)** | **Sequence name** |
|---|---|---|
| 1 | 2 | H7N9 HA2 extracellular domain |
| 3 | 4 | Collagen multimerized motif (C) |
| 5 | 6 | Foldon multimerized motif (F) |
| 7 | 8 | Tetrabrachion helical peptide multimerized motif |
| 9 | 10 | GCN4 multimerized motif |
| 11 | 12* | T-cell antigen (T) |
| 13 | 14 | M2e of certain H1N1/H3N2 subtypes of human influenza |
| 15 | 16 | M2e of certain H1N1/H3N2 subtypes of human influenza |
| 17 | 18 | M2e of avian influenza H7N9 subtype |
| 19 | 20 | M2e of avian influenza subtypes such as H5N1/H9N2 |
| 21 | 22 | 4M2e (M4) comprising four tandem repeats of M2e |
| 23 | 24 | 8M2e (M8) comprising eight tandem repeats of M2e |
| 25 | 26* | 8M2e-HA2-collagen (M8HC) |
| 27 | 28* | 4M2e-HA2-foldon (M4HF) |
| 29 | 30* | 4M2e-H1N1 HA2-foldon (M4H1F) |
| 31 | 32* | 4M2e-H3N2 HA2-foldon (M4H3F) |
| 33 | 34* | 2M2e- HA2-foldon (M2HF) |
| 35 | 36* | 4M2e- HA2 (M4H) |
| 37 | 38* | HA2-foldon (HF) |
| 39 | 40* | 4M2e-HA2-collagen (M4HC) |
| | 41* | IRES nucleotide sequence |
| | 42* | 4M2e-HA2-foldon-IRES-T-DHFR (M4HF-T) |
| 43 | 44* | DHFR |
| 45 | | Peptide linker GGGGS |
| 46 | | Peptide linker GSAGSAAGSGEF |
| 47 | | Peptide linker EAAAK |
| 48 | | Peptide linker EFPKPSTPPGSSGGAP |
| 49 | | Peptide linker KESGSVSSEQLAQFRSLD |
| 50 | | Peptide linker EGKSSGSGSESKST |
| 51 | | T cell antigen 1 (SEQ ID NO: 1 of CN201880001963.X) |
| 52 | | T cell antigen 2 (SEQ ID NO: 2 of CN201880001963.X) |

| | | |
|---|---|---|
| **Note:** * indicates that the sequence is the one used in the example. | | |

### Specific sequences

SEQ ID NO:1 (amino acid sequence of H7N9 HA2 extracellular domain) 
GLFGAIAGFIENGWEGLIDGWYGFRHQNAQGEGTAADYKSTQSAIDQITGKLNRLIEKTNQQFELIDNE
FNEVEKQIGNVIRWTRDSITEVWSYNAELLVAMENQHTIDLADSEMDKLYERVKRQLRENAEEDGTG
CFEIFHKCDDDCMASIRHNTYDHSKYREEAMQNRIQIDPVKLSSGYKDV
SEQ ID NO:2 (nucleotide sequence of H7N9 HA2 extracellular domain) 
GGCCTTTTTGGAGCAATCGCTGGATTCATTGAAAATGGGTGGGAGGGCCTCATCGACGGGTGGTAC
GGATTCAGGCACCAAAACGCCCAGGGAGAAGGGACCGCAGCTGACTACAAGTCAACACAGTCAG
CTATTGATCAGATTACCGGGAAGCTGAATAGACTGATTGAAAAGACCAACCAGCAGTTCGAGCTG
ATCGATAACGAGTTTAACGAGGTCGAAAAGCAGATAGGAAATGTAATCAGGTGGACACGCGATTC
TATCACCGAAGTCTGGTCCTACAATGCCGAGCTGCTGGTTGCAATGGAGAACCAACACACTATCGA
CCTTGCCGATTCCGAAATGGACAAACTGTATGAACGCGTGAAGCGGCAGCTCAGGGAAAACGCTG
AGGAAGACGGTACCGGCTGTTTCGAAATTTTCCACAAGTGCGATGATGACTGTATGGCCAGCATCA
GGCACAACACCTACGATCATTCCAAGTATAGAGAGGAGGCCATGCAGAACCGGATTCAGATCGAT
CCAGTGAAACTGTCATCCGGCTACAAGGACGTG
SEQ ID NO:3 (Collagen amino acid sequence)
   DKIREVPEGWLIFVAEREELYVRVRNGFRKVLLEARTALPRGTGNE
SEQ ID NO:4 (Collagen nucleotide sequence) 
GACAAGATCAGGGAGGTGCCTGAGGGCTGGCTCATCTTCGTGGCCGAGCGGGAGGAGCTGTACGT
GCGGGTGAGAAACGGCTTCAGAAAGGTGCTCTTAGAAGCCCGGACAGCCCTGCCACGGGGCACAG
GCAACGAG
SEQ ID NO:5 (Foldon amino acid sequence)
   GYIPEAPRDGQAYVRKDGEWVLLSTFL
SEQ ID NO:6 (Foldon nucleotide sequence) 
GGATACATTCCAGAGGCCCCCAGAGACGGACAGGCTTATGTGAGAAAGGACGGAGAGTGGGTGCT
GCTGAGTACATTTCTG
SEQ ID NO:7 (Amino acid sequence of tetrabrachion helical peptide)
   IINETADDIVYRLTVIIDDRYESLKNLITLRADRLEMIINDNVSTILAS
SEQ ID NO:8 (Nucleotide sequence of tetrabrachion helical peptide) 
ATCATCAACGAGACTGCTGACGACATCGTGTACCGTCTGACCGTGATCATCGACGACCGCTACGAG
TCCCTGAAGAACCTGATCACCCTGCGTGCTGACCGTCTGGAAATGATTATCAACGACAACGTGTCC
ACCATCCTGGCTTCC
SEQ ID NO:9 (GCN4 amino acid sequence)
   EELLSKNYHLENEVARLKK
SEQ ID NO:10 (GCN4 nucleotide sequence)
   GAAGAATTGCTTTCGAAAAATTATCACTTGGAAAATGAGGTTGCCAGATTAAAGAAA
SEQ ID NO:11 (Amino acid sequence of T cell antigen)* 
MDVNPTLLFLKVPAQNAISTTFPYTGDPPYSHGTGTGYTMDTVNRTHQYSEKGKWTTNTETGAPQLNP
IDGPLPEDNEPSGYAQHFQRKRRVRDNMTKKMVTQRTIGKKKQRLNKRGYLIRALTLNTMTKDAERG
KLKRRAIATPGMQIRGFVYFVETLARSICEKLEQSGLPVGGNEKKAKLANVVRKMMTNSQDTEISFTIT
GDNTKWNENQNPRMFLAMITYITKNQPEWFRNILSIAPIMFSNKMARLGKGYMFESKRMKLRTQIPAE
MLASIDLKYFNESTKKKIEKIRPLLIDGTASLSPGMMMGMFNMLSTVLGVSILNLGQKKYTKTTYWWD
GLQSSDDFALIVNAPNHEGIQAGVDRFYRTCKLVGINMSKKKSYINKTGTFEFTSFFYRYGFVANFSME
LPSFGVSGVNESADMSIGVTVIKNNMINNDLGPATAQMALQLFIKDYRYTYRCHRGDTQIQTRRSFELK
KLWDQTQSKAGLLVSDGGPNLYNIRNLHIPEVCLKWELMDEDYRGRLCNPLNPFVSHKEIESVNNAVV
MPAHGPAKSMEYDAVATTHSWIPKRNRSILNTSQRGILEDEQMYQKCCNLFEKFFPSSSYRRPVGISSM
VEAMVSRARIDARIDFESGRIKKEEFSEIMKICSTIEELRRQKGGGGSGGGGSMRRNYFTAEVSHCRATE
YIMKGVYINTALLNASCAAMDDFQLIPMISKCRTKEGRRKTNLYGFIIKGRSHLRNDTDVVNFVSMEFS
LTDPRLEMFLYVRTNGTSKIKMKWGMEMRRCLLQSLQQIESMIEAESSVKEKDMTKEFFENKSETWPI
GESPKGVEEGSIGKVCRTLLAKSVFNFDLGGLYEAIEECLINDPWVLLNASWFNSFLTHALKGSGGSGM
TKITLSPQNFRIQKQETTLLKEKSTEKNSLAKSILAVKNHFIELRSKLSERFISHKNTESSATHFHRGSASE
GRAVLTNKVVKDFMLQTLNDIDIRGSA
SEQ ID NO:12 (Nucleotide sequence of T cell antigen) 
ATGGACGTGAACCCCACCCTGCTGTTTCTGAAGGTGCCCGCTCAGAACGCCATCTCCACCACATTC
CCCTACACCGGCGACCCTCCTTACTCCCATGGCACAGGAACCGGCTACACCATGGACACCGTGAAC
AGAACCCACCAGTACAGCGAGAAAGGCAAATGGACCACCAACACCGAAACCGGCGCCCCTCAGC
TGAACCCTATCGATGGACCCCTGCCCGAAGACAACGAACCCTCAGGATACGCCCAGCACTTCCAG
AGAAAAAGAAGAGTGAGAGACAACATGACCAAAAAGATGGTGACCCAGAGAACCATCGGCAAGA
AGAAACAGAGACTGAACAAGAGAGGATACCTGATCAGAGCCCTGACCCTGAACACCATGACCAA
GGACGCCGAGAGAGGCAAGCTGAAGAGAAGAGCCATCGCCACCCCCGGAATGCAGATCAGAGGA
TTCGTGTACTTCGTGGAGACCCTGGCCAGAAGCATCTGCGAAAAGCTGGAGCAGAGCGGACTGCC
AGTGGGAGGAAACGAAAAAAAGGCCAAGCTGGCCAACGTGGTGAGAAAGATGATGACCAACAGC
CAGGACACCGAGATCAGCTTCACCATCACCGGCGACAACACCAAGTGGAACGAGAACCAGAACCC
CAGAATGTTCCTGGCCATGATCACCTACATCACCAAGAACCAGCCCGAGTGGTTCAGAAACATCCT
GAGCATCGCCCCCATCATGTTCAGCAACAAGATGGCCAGACTGGGCAAGGGCTACATGTTCGAGA
GCAAGAGAATGAAGCTGAGAACCCAGATCCCCGCCGAGATGCTGGCCAGCATCGACCTGAAGTAC
TTCAACGAGAGCACCAAGAAGAAGATCGAGAAGATCAGACCCCTGCTGATCGACGGCACCGCCAG
CCTGAGCCCCGGCATGATGATGGGCATGTTCAACATGCTGAGCACCGTGCTGGGCGTGAGCATCCT
GAACCTGGGCCAGAAGAAGTACACCAAGACCACCTACTGGTGGGACGGCCTGCAGAGCAGCGAC
GACTTCGCCCTGATCGTGAACGCCCCCAACCACGAGGGCATCCAGGCCGGCGTGGACAGATTCTA
CAGAACCTGCAAGCTGGTGGGCATCAACATGAGCAAGAAGAAGAGCTACATCAACAAGACCGGC
ACCTTCGAGTTCACCAGCTTCTTCTACAGATACGGCTTCGTGGCCAACTTCAGCATGGAGCTGCCC
AGCTTCGGCGTGAGCGGCGTGAACGAGAGCGCCGACATGAGCATCGGCGTGACCGTGATCAAGAA
CAACATGATCAACAACGACCTGGGCCCCGCCACCGCCCAGATGGCCCTGCAGCTGTTCATCAAGG
ACTACAGATACACCTACAGATGCCACAGAGGCGACACCCAGATCCAGACCAGAAGAAGCTTCGAG
CTGAAGAAGCTGTGGGACCAGACCCAGAGCAAGGCCGGCCTGCTGGTGAGCGACGGCGGCCCCA
ACCTGTACAACATCAGAAACCTGCACATCCCCGAGGTGTGCCTGAAGTGGGAGCTGATGGACGAG
GACTACAGAGGCAGACTGTGCAACCCCCTGAACCCCTTCGTGAGCCACAAGGAGATCGAGAGCGT
GAACAACGCCGTGGTGATGCCCGCCCACGGCCCCGCCAAGAGCATGGAGTACGACGCCGTGGCCA
CCACCCACAGCTGGATTCCTAAGAGAAACAGAAGCATCCTGAACACCAGCCAGAGAGGCATCCTG
GAGGACGAGCAGATGTACCAGAAGTGCTGCAACCTGTTCGAGAAGTTCTTCCCCAGCAGCAGCTA
CAGAAGACCCGTGGGCATCAGCAGCATGGTGGAGGCCATGGTGAGCAGAGCCAGAATCGACGCC
AGAATCGACTTCGAGAGCGGCAGAATCAAGAAGGAGGAGTTCAGCGAGATCATGAAGATCTGCA
 
GCACCATCGAGGAGCTGAGAAGACAGAAGGCGGCGGCAGCGGCGGCGGCAGCATGAG
AAGAAACTACTTCACCGCCGAGGTGAGCCACTGCAGAGCCACCGAGTACATCATGAAGGGCGTGT
ACATCAACACCGCCCTGCTGAACGCCAGCTGCGCCGCCATGGACGACTTCCAGCTGATCCCCATGA
TCAGCAAGTGCAGAACCAAGGAGGGCAGAAGAAAGACCAACCTGTACGGCTTCATCATCAAGGG
CAGAAGCCACCTGAGAAACGACACCGACGTGGTGAACTTCGTGAGCATGGAGTTCAGCCTGACCG
ACCCCAGACTGGAGATGTTCCTGTACGTGAGAACCAACGGCACCAGCAAGATCAAGATGAAGTGG
GGCATGGAGATGAGAAGATGCCTGCTGCAGAGCCTGCAGCAGATCGAGAGCATGATCGAGGCCG
AGAGCAGCGTGAAGGAGAAGGACATGACCAAGGAGTTCTTCGAGAACAAGAGCGAGACCTGGCC
CATCGGCGAGAGCCCCAAGGGCGTGGAGGAGGGCAGCATCGGCAAGGTGTGCAGAACCCTGCTG
GCCAAGAGCGTGTTCAACTTCGACCTGGGCGGCCTGTACGAGGCCATCGAGGAGTGCCTGATCAA
CGACCCCTGGGTGCTGCTGAACGCCAGCTGGTTCAACAGCTTCCTGACCCACGCCCTGAAGGGCAG
CGGCGGCAGCGGCATGACCAAGATCACCCTGAGCCCCCAGAACTTCAGAATCCAGAAGCAGGAGA
CCACCCTGCTGAAGGAGAAGAGCACCGAGAAGAACAGCCTGGCCAAGAGCATCCTGGCCGTGAA
GAACCACTTCATCGAGCTGAGAAGCAAGCTGAGCGAGAGATTCATCAGCCACAAGAACACCGAGA
GCAGCGCCACCCACTTCCACAGAGGCAGCGCCAGCGAGGGCAGAGCCGTGCTGACCAACAAGGTG
GTGAAGGACTTCATGCTGCAGACCCTGAACGACATCGACATCAGAGGCAGCGCC
SEQ ID NO:13 (M2e amino acid sequence derived from some H1N1/H3N2 subtypes that infect humans)
   SLLTEVETPIRNEWGCRCNGSSDP
SEQ ID NO:14 (M2e nucleotide sequence derived from some H1N1/H3N2 subtypes that infect humans) 
TCCCTCCTGACCGAGGTGGAGACACCTATCCGGAACGAGTGGGGGTGTAGATGCAACGGCAGCAG
CGACCCA
SEQ ID NO : 15 (M2e amino acid sequence derived from other H1N1/H3N2 subtypes that infect humans)
   SLLTEVETPIRNEWGCRCNDSSDP
SEQ ID NO:16 (M2e nucleotide sequence derived from other H1N1/H3N2 subtypes that infect humans) 
AGCCTCCTCACAGAGGTGGAGACACCAATCAGGAACGAGTGGGGATGCAGATGCAACGACTCTAG
CGACCCA
SEQ ID NO:17 (M2e amino acid sequence derived from an H7N9 subtype whose natural host is avian species)
   SLLTEVETPTRTGWECNCSGSSEP
SEQ ID NO:18 (M2e nucleotide sequence derived from an H7N9 subtype whose natural host is avian species) 
AGTCTCCTCACAGAGGTGGAGACACCTACCAGGACCGGCTGGGAGTGCAACTGCTCTGGCAGCTC
TGAGCCA
SEQ ID NO:19 (M2e amino acid sequence derived from subtypes such as H5N1/H9N2 whose natural hosts are avian species)
   SLLTEVETPTRNEWECKCSDSSDP
SEQ ID NO:20 (M2e nucleotide sequence derived from subtypes such as H5N1/H9N2 whose natural hosts are avian species) 
TCTCTCCTCACAGAGGTGGAGACACCTACAAGAAACGAGTGGGAGTGCAAGTGCTCCGACTCTTC
CGACCCA
SEQ ID NO:21 (4M2e amino acid sequence) 
SLLTEVETPIRNEWGCRCNGSSDPAAASLLTEVETPIRNEWGCRCNDSSDPAAASLLTEVETPTRTGWE
CNCSGSSEPAAASLLTEVETPTRNEWECKCSDSSDP
SEQ ID NO:22 (4M2e nucleotide sequence) 
TCCCTCCTGACCGAGGTGGAGACACCTATCCGGAACGAGTGGGGGTGTAGATGCAACGGCAGCAG
CGACCCAGCTGCCGCAAGCCTCCTCACAGAGGTGGAGACACCAATCAGGAACGAGTGGGGATGCA
GATGCAACGACTCTAGCGACCCAGCTGCCGCAAGTCTCCTCACAGAGGTGGAGACACCTACCAGG
ACCGGCTGGGAGTGCAACTGCTCTGGCAGCTCTGAGCCAGCTGCCGCTTCTCTCCTCACAGAGGTG
GAGACACCTACAAGAAACGAGTGGGAGTGCAAGTGCTCCGACTCTTCCGACCCA
SEQ ID NO:23 (8M2e amino acid sequence)* 
SLLTEVETPIRNEWGCRCNGSSDPAAASLLTEVETPIRNEWGCRCNDSSDPAAASLLTEVETPTRTGWE
CNCSGSSEPAAASLLTEVETPTRNEWECKCSDSSDPAAASLLTEVETPIRNEWGCRCNGSSDPAAASLL
TEVETPIRNEWGCRCNDSSDPAAASLLTEVETPTRTGWECNCSGSSEPAAASLLTEVETPTRNEWECKC
SDSSDP
SEQ ID NO:24 (8M2e nucleotide sequence) 
TCCCTCCTGACCGAGGTGGAGACACCTATCCGGAACGAGTGGGGGTGTAGATGCAACGGCTCTTCT
GACCCCGCCGCCGCTTCCCTCCTCACAGAGGTGGAGACCCCTATCAGGAACGAGTGGGGTTGCAG
ATGCAACGACTCTAGCGACCCAGCCGCCGCTTCTCTCCTGACCGAGGTGGAGACACCAACAAGAA
CCGGCTGGGAGTGCAACTGCTCTGGCTCTTCTGAGCCAGCTGCCGCTTCCCTCCTGACCGAGGTGG
AGACACCTACAAGAAACGAGTGGGAGTGCAAGTGCTCCGACTCTAGCGACCCCGCCGCCGCTTCC
CTGCTCACAGAGGTGGAGACACCAATCCGGAACGAGTGGGGTTGTAGATGCAACGGCAGCAGCGA
CCCAGCTGCCGCAAGCCTCCTCACAGAGGTGGAGACACCAATCAGGAACGAGTGGGGATGCAGAT
GCAACGACTCTAGCGACCCAGCTGCCGCAAGTCTCCTCACAGAGGTGGAGACACCTACCAGGACC
GGCTGGGAGTGCAACTGCTCTGGCAGCTCTGAGCCAGCTGCCGCTTCTCTCCTCACAGAGGTGGAG
ACACCTACAAGAAACGAGTGGGAGTGCAAGTGCTCCGACTCTTCCGACCCA
SEQ ID NO:25 (Amino acid sequence of 8M2e-HA2-collagen)* 
SLLTEVETPIRNEWGCRCNGSSDPAAASLLTEVETPIRNEWGCRCNDSSDPAAASLLTEVETPTRTGWE
CNCSGSSEPAAASLLTEVETPTRNEWECKCSDSSDPAAASLLTEVETPIRNEWGCRCNGSSDPAAASLL
TEVETPIRNEWGCRCNDSSDPAAASLLTEVETPTRTGWECNCSGSSEPAAASLLTEVETPTRNEWECKC
SDSSDPSAPGTPGLFGAIAGFIENGWEGLIDGWYGFRHQNAQGEGTAADYKSTQSAIDQITGKLNRLIE
KTNQQFELIDNEFNEVEKQIGNVIRWTRDSITEVWSYNAELLVAMENQHTIDLADSEMDKLYERVKRQ
LRENAEEDGTGCFEIFHKCDDDCMASIRHNTYDHSKYREEAMQNRIQIDPVKLSSGYKDVDKIREVPEG
WLIFVAEREELYVRVRNGFRKVLLEARTALPRGTGNE
SEQ ID NO:26 (Nucleotide sequence of 8M2e-HA2-collagen) 
TCCCTCCTGACCGAGGTGGAGACACCTATCCGGAACGAGTGGGGGTGTAGATGCAACGGCTCTTCT
GACCCCGCCGCCGCTTCCCTCCTCACAGAGGTGGAGACCCCTATCAGGAACGAGTGGGGTTGCAG
ATGCAACGACTCTAGCGACCCAGCCGCCGCTTCTCTCCTGACCGAGGTGGAGACACCAACAAGAA
CCGGCTGGGAGTGCAACTGCTCTGGCTCTTCTGAGCCAGCTGCCGCTTCCCTCCTGACCGAGGTGG
AGACACCTACAAGAAACGAGTGGGAGTGCAAGTGCTCCGACTCTAGCGACCCCGCCGCCGCTTCC
CTGCTCACAGAGGTGGAGACACCAATCCGGAACGAGTGGGGTTGTAGATGCAACGGCAGCAGCGA
CCCAGCTGCCGCAAGCCTCCTCACAGAGGTGGAGACACCAATCAGGAACGAGTGGGGATGCAGAT
GCAACGACTCTAGCGACCCAGCTGCCGCAAGTCTCCTCACAGAGGTGGAGACACCTACCAGGACC
GGCTGGGAGTGCAACTGCTCTGGCAGCTCTGAGCCAGCTGCCGCTTCTCTCCTCACAGAGGTGGAG
ACACCTACAAGAAACGAGTGGGAGTGCAAGTGCTCCGACTCTTCCGACCCAAGCGCCCCAGGCAC
ACCTGGCCTTTTTGGAGCAATCGCTGGATTCATTGAAAATGGGTGGGAGGGCCTCATCGACGGGTG
GTACGGATTCAGGCACCAAAACGCCCAGGGAGAAGGGACCGCAGCTGACTACAAGTCAACACAG
TCAGCTATTGATCAGATTACCGGGAAGCTGAATAGACTGATTGAAAAGACCAACCAGCAGTTCGA
GCTGATCGATAACGAGTTTAACGAGGTCGAAAAGCAGATAGGAAATGTAATCAGGTGGACACGCG
ATTCTATCACCGAAGTCTGGTCCTACAATGCCGAGCTGCTGGTTGCAATGGAGAACCAACACACTA
TCGACCTTGCCGATTCCGAAATGGACAAACTGTATGAACGCGTGAAGCGGCAGCTCAGGGAAAAC
GCTGAGGAAGACGGTACCGGCTGTTTCGAAATTTTCCACAAGTGCGATGATGACTGTATGGCCAGC
ATCAGGCACAACACCTACGATCATTCCAAGTATAGAGAGGAGGCCATGCAGAACCGGATTCAGAT
CGATCCAGTGAAACTGTCATCCGGCTACAAGGACGTGGACAAGATCAGGGAGGTGCCTGAGGGCT
GGCTCATCTTCGTGGCCGAGCGGGAGGAGCTGTACGTGCGGGTGAGAAACGGCTTCAGAAAGGTG
CTCTTAGAAGCCCGGACAGCCCTGCCACGGGGCACAGGCAACGAG
SEQ ID NO:27 (Amino acid sequence of 4M2e-HA2-foldon)* 
SLLTEVETPIRNEWGCRCNGSSDPAAASLLTEVETPIRNEWGCRCNDSSDPAAASLLTEVETPTRTGWE
CNCSGSSEPAAASLLTEVETPTRNEWECKCSDSSDPSAPGTPGLFGAIAGFIENGWEGLIDGWYGFRHQ
NAQGEGTAADYKSTQSAIDQITGKLNRLIEKTNQQFELIDNEFNEVEKQIGNVIRWTRDSITEVWSYNA
ELLVAMENQHTIDLADSEMDKLYERVKRQLRENAEEDGTGCFEIFHKCDDDCMASIRHNTYDHSKYR
EEAMQNRIQIDPVKLSSGYKDVLETGGGYIPEAPRDGQAYVRKDGEWVLLSTFL
SEQ ID NO:28 (Nucleotide sequence of 4M2e-HA2-foldon) 
TCCCTCCTGACCGAGGTGGAGACACCTATCCGGAACGAGTGGGGGTGTAGATGCAACGGCAGCAG
CGACCCAGCTGCCGCAAGCCTCCTCACAGAGGTGGAGACACCAATCAGGAACGAGTGGGGATGCA
GATGCAACGACTCTAGCGACCCAGCTGCCGCAAGTCTCCTCACAGAGGTGGAGACACCTACCAGG
ACCGGCTGGGAGTGCAACTGCTCTGGCAGCTCTGAGCCAGCTGCCGCTTCTCTCCTCACAGAGGTG
GAGACACCTACAAGAAACGAGTGGGAGTGCAAGTGCTCCGACTCTTCCGACCCAAGCGCCCCAGG
CACACCTGGCCTTTTTGGAGCAATCGCTGGATTCATTGAAAATGGGTGGGAGGGCCTCATCGACGG
GTGGTACGGATTCAGGCACCAAAACGCCCAGGGAGAAGGGACCGCAGCTGACTACAAGTCAACA
CAGTCAGCTATTGATCAGATTACCGGGAAGCTGAATAGACTGATTGAAAAGACCAACCAGCAGTT
CGAGCTGATCGATAACGAGTTTAACGAGGTCGAAAAGCAGATAGGAAATGTAATCAGGTGGACAC
GCGATTCTATCACCGAAGTCTGGTCCTACAATGCCGAGCTGCTGGTTGCAATGGAGAACCAACACA
CTATCGACCTTGCCGATTCCGAAATGGACAAACTGTATGAACGCGTGAAGCGGCAGCTCAGGGAA
AACGCTGAGGAAGACGGTACCGGCTGTTTCGAAATTTTCCACAAGTGCGATGATGACTGTATGGCC
AGCATCAGGCACAACACCTACGATCATTCCAAGTATAGAGAGGAGGCCATGCAGAACCGGATTCA
GATCGATCCAGTGAAACTGTCATCCGGCTACAAGGACGTGCTCGAGACCGGCGGCGGATACATTC
CAGAGGCCCCCAGAGACGGACAGGCTTATGTGAGAAAGGACGGAGAGTGGGTGCTGCTGAGTAC
ATTTCTG
SEQ ID NO:29 (Amino acid sequence of 4M2e-H1N1 HA2-foldon)* 
SLLTEVETPIRNEWGCRCNGSSDPAAASLLTEVETPIRNEWGCRCNDSSDPAAASLLTEVETPTRTGWE
CNCSGSSEPAAASLLTEVETPTRNEWECKCSDSSDPSAPGTPCTGLFGAIAGFIEGGWTGMIDGWYGYH
HQNEQGSGYAADQKSTQNAINGITNKVNSVIEKMNIQFTAVGKEFNKLEKRMENLNKKVDDGFLDIW
TYNAELLVLLENERTLDFHDSNVKNLYEKVKSQLKNNAKEIGNGCFEFYHKCDNECMESVRNGTYDY
PKYSEESKLNREKVDGVKLESMGIYQILETGGGYIPEAPRDGQAYVRKDGEWVLLSTFL
SEQ ID NO:30 (Nucleotide sequence of 4M2e-H1N1 HA2-foldon) 
TCCCTCCTGACCGAGGTGGAGACACCTATCCGGAACGAGTGGGGGTGTAGATGCAACGGCAGCAG
CGACCCAGCTGCCGCAAGCCTCCTCACAGAGGTGGAGACACCAATCAGGAACGAGTGGGGATGCA
GATGCAACGACTCTAGCGACCCAGCTGCCGCAAGTCTCCTCACAGAGGTGGAGACACCTACCAGG
ACCGGCTGGGAGTGCAACTGCTCTGGCAGCTCTGAGCCAGCTGCCGCTTCTCTCCTCACAGAGGTG
GAGACACCTACAAGAAACGAGTGGGAGTGCAAGTGCTCCGACTCTTCCGACCCAAGCGCCCCAGG
CACACCTTGTACAGGCCTGTTCGGCGCGATCGCGGGCTTCATCGAAGGCGGCTGGACCGGCATGAT
CGATGGCTGGTACGGCTACCACCACCAGAACGAACAGGGCAGCGGCTACGCGGCTGATCAGAAAA
GCACCCAGAACGCGATCAACGGCATCACCAACAAAGTTAACAGCGTTATCGAAAAAATGAACATC
CAGTTCACCGCGGTTGGTAAAGAATTCAACAAACTGGAAAAGCGTATGGAAAACCTGAACAAAAA
AGTTGATGATGGCTTCCTGGATATCTGGACCTACAACGCGGAACTGCTGGTTCTGCTGGAAAACGA
ACGTACCCTGGATTTCCACGATAGCAACGTGAAAAACCTGTACGAAAAAGTTAAAAGCCAGCTGA
AAAACAACGCGAAAGAAATCGGTAACGGCTGCTTCGAATTCTACCACAAATGCGATAACGAATGT
ATGGAAAGCGTTCGTAACGGTACTTATGATTACCCTAAGTATTCCGAAGAATCTAAACTGAACCGT
GAAAAAGTTGACGGCGTCAAATTAGAATCTATGGGTATCTATCAGATCCTCGAGACCGGCGGCGG
ATACATTCCAGAGGCCCCCAGAGACGGACAGGCTTATGTGAGAAAGGACGGAGAGTGGGTGCTGC
TGAGTACATTTCTG
SEQ ID NO:31 (Amino acid sequence of 4M2e-H3N2 HA2-foldon)* 
SLLTEVETPIRNEWGCRCNGSSDPAAASLLTEVETPIRNEWGCRCNDSSDPAAASLLTEVETPTRTGWE
CNCSGSSEPAAASLLTEVETPTRNEWECKCSDSSDPSAPGTPCTGLFGAIAGFIENGWEGMIDGWYGFR
HQNSEGTGQAADLKSTQAAIDQINGKLNRVIEKTNEKFHQIEKEFSEVEGRIQDLEKYVEDTKIDLWSY
NAELLVALENQHTIDLTDSEMNKLFEKTRRQLRENAEDMGNGCFKIYHKCDNACIESIRNGTYDHDVY
 RDEALNNRFQIKGVELKSGYKDLETGGGYIPEAPRDGQAYVRKDGEWVLLSTFL
SEQ ID NO:32 (Nucleotide sequence of 4M2e-H3N2 HA2-foldon) 
TCCCTCCTGACCGAGGTGGAGACACCTATCCGGAACGAGTGGGGGTGTAGATGCAACGGCAGCAG
CGACCCAGCTGCCGCAAGCCTCCTCACAGAGGTGGAGACACCAATCAGGAACGAGTGGGGATGCA
GATGCAACGACTCTAGCGACCCAGCTGCCGCAAGTCTCCTCACAGAGGTGGAGACACCTACCAGG
ACCGGCTGGGAGTGCAACTGCTCTGGCAGCTCTGAGCCAGCTGCCGCTTCTCTCCTCACAGAGGTG
GAGACACCTACAAGAAACGAGTGGGAGTGCAAGTGCTCCGACTCTTCCGACCCAAGCGCCCCAGG
CACACCTTGTACAGGCCTGTTCGGGGCGATAGCAGGCTTCATTGAAAACGGGTGGGAGGGTATGA
TTGATGGCTGGTACGGCTTCCGTCACCAGAACAGCGAAGGTACCGGCCAGGCGGCGGATCTGAAA
AGCACCCAGGCGGCGATCGATCAGATCAACGGTAAACTGAACCGTGTTATCGAAAAAACCAACGA
AAAATTCCACCAGATCGAAAAAGAATTCAGCGAAGTTGAAGGCCGTATCCAGGATCTGGAAAAAT
ACGTTGAAGATACCAAAATCGATCTGTGGAGCTACAACGCGGAACTGCTGGTTGCGCTGGAAAAC
CAGCACACGATCGATCTGACCGATAGCGAAATGAACAAACTGTTCGAAAAAACCCGTCGTCAGCT
GCGTGAAAACGCGGAAGATATGGGTAACGGTTGCTTCAAAATCTACCACAAATGCGATAACGCGT
GCATCGAAAGCATCCGTAACGGCACCTACGATCACGATGTTTACCGTGATGAAGCGCTGAACAAC
CGTTTCCAGATCAAAGGCGTTGAACTGAAAAGCGGCTACAAAGATCTCGAGACCGGCGGCGGATA
CATTCCAGAGGCCCCCAGAGACGGACAGGCTTATGTGAGAAAGGACGGAGAGTGGGTGCTGCTGA
GTACATTTCTG
SEQ ID NO:33 (Amino acid sequence of 2M2e- HA2-foldon)* 
SLLTEVETPIRNEWGCRCNGSSDPAASLLTEVETPIRNEWGCRCNDSSDPRTSAPGGLFGAIAGFIENG
WEGLIDGWYGFRHQNAQGEGTAADYKSTQSAIDQITGKLNRLIEKTNQQFELIDNEFNEVEKQIGNVIR
WTRDSITEVWSYNAELLVAMENQHTIDLADSEMDKLYERVKRQLRENAEEDGTGCFEIFHKCDDDCM
ASIRHNTYDHSKYREEAMQNRIQIDPVKLSSGYKDVLETGGGYIPEAPRDGQAYVRKDGEWVLLSTFL
SEQ ID NO:34 (Nucleotide sequence of 2M2e- HA2-foldon) 
TCCCTCCTGACCGAGGTGGAGACACCTATCCGGAACGAGTGGGGGTGTAGATGCAACGGCAGCAG
CGACCCAGCTGCCGCAAGCCTCCTCACAGAGGTGGAGACACCAATCAGGAACGAGTGGGGATGCA
GATGCAACGACTCTAGCGACCCACGTACGAGCGCCCCAGGCGGCCTTTTTGGAGCAATCGCTGGA
TTCATTGAAAATGGGTGGGAGGGCCTCATCGACGGGTGGTACGGATTCAGGCACCAAAACGCCCA
GGGAGAAGGGACCGCAGCTGACTACAAGTCAACACAGTCAGCTATTGATCAGATTACCGGGAAGC
TGAATAGACTGATTGAAAAGACCAACCAGCAGTTCGAGCTGATCGATAACGAGTTTAACGAGGTC
GAAAAGCAGATAGGAAATGTAATCAATTGGACACGCGATTCTATCACCGAAGTCTGGTCCTACAA
TGCCGAGCTGCTGGTTGCAATGGAGAACCAACACACTATCGACCTTGCCGATTCCGAAATGGACA
AACTGTATGAACGCGTGAAGCGGCAGCTCAGGGAAAACGCTGAGGAAGACGGTACCGGCTGTTTC
GAAATTTTCCACAAGTGCGATGATGACTGTATGGCCAGCATCAGGAATAACACCTACGATCATTCC
AAGTATAGAGAGGAGGCCATGCAGAACCGGATTCAGATCGATCCAGTGAAACTGTCATCCGGCTA
CAAGGACGTGCTCGAGACCGGCGGCGGATACATTCCAGAGGCCCCCAGAGACGGACAGGCTTATG
TGAGAAAGGACGGAGAGTGGGTGCTGCTGAGTACATTTCTG
SEQ ID NO:35 (4M2e-HA2 amino acid sequence)* 
SLLTEVETPIRNEWGCRCNGSSDPAAASLLTEVETPIRNEWGCRCNDSSDPAAASLLTEVETPTRTGWE
CNCSGSSEPAAASLLTEVETPTRNEWECKCSDSSDPSAPGTPGLFGAIAGFIENGWEGLIDGWYGFRHQ
NAQGEGTAADYKSTQSAIDQITGKLNRLIEKTNQQFELIDNEFNEVEKQIGNVIRWTRDSITEVWSYNA
ELLVAMENQHTIDLADSEMDKLYERVKRQLRENAEEDGTGCFEIFHKCDDDCMASIRHNTYDHSKYR
EEAMQNRIQIDPVKLSSGYKDV
SEQ ID NO:36 (4M2e-HA2 nucleotide sequence) 
TCCCTCCTGACCGAGGTGGAGACACCTATCCGGAACGAGTGGGGGTGTAGATGCAACGGCAGCAG
CGACCCAGCTGCCGCAAGCCTCCTCACAGAGGTGGAGACACCAATCAGGAACGAGTGGGGATGCA
GATGCAACGACTCTAGCGACCCAGCTGCCGCAAGTCTCCTCACAGAGGTGGAGACACCTACCAGG
ACCGGCTGGGAGTGCAACTGCTCTGGCAGCTCTGAGCCAGCTGCCGCTTCTCTCCTCACAGAGGTG
GAGACACCTACAAGAAACGAGTGGGAGTGCAAGTGCTCCGACTCTTCCGACCCAAGCGCCCCAGG
CACACCTGGCCTTTTTGGAGCAATCGCTGGATTCATTGAAAATGGGTGGGAGGGCCTCATCGACGG
 
GTGGTACGGATTCAGGCACCAAAACGCCCAGGGAGAAGGGACCGCAGCTGACTACAAGTCAACA
CAGTCAGCTATTGATCAGATTACCGGGAAGCTGAATAGACTGATTGAAAAGACCAACCAGCAGTT
CGAGCTGATCGATAACGAGTTTAACGAGGTCGAAAAGCAGATAGGAAATGTAATCAGGTGGACAC
GCGATTCTATCACCGAAGTCTGGTCCTACAATGCCGAGCTGCTGGTTGCAATGGAGAACCAACACA
CTATCGACCTTGCCGATTCCGAAATGGACAAACTGTATGAACGCGTGAAGCGGCAGCTCAGGGAA
AACGCTGAGGAAGACGGTACCGGCTGTTTCGAAATTTTCCACAAGTGCGATGATGACTGTATGGCC
AGCATCAGGCACAACACCTACGATCATTCCAAGTATAGAGAGGAGGCCATGCAGAACCGGATTCA
GATCGATCCAGTGAAACTGTCATCCGGCTACAAGGACGTG
SEQ ID NO:37 (HA2-foldon amino acid sequence)* 
GLFGAIAGFIENGWEGLIDGWYGFRHQNAQGEGTAADYKSTQSAIDQITGKLNRLIEKTNQQFELIDNE
FNEVEKQIGNVIRWTRDSITEVWSYNAELLVAMENQHTIDLADSEMDKLYERVKRQLRENAEEDGTG
CFEIFHKCDDDCMASIRHNTYDHSKYREEAMQNRIQIDPVKLSSGYKDVLETGGGYIPEAPRDGQAYV
RKDGEWVLLSTFL
SEQ ID NO:38 (HA2-foldon nucleotide sequence) 
GGCCTTTTTGGAGCAATCGCTGGATTCATTGAAAATGGGTGGGAGGGCCTCATCGACGGGTGGTAC
GGATTCAGGCACCAAAACGCCCAGGGAGAAGGGACCGCAGCTGACTACAAGTCAACACAGTCAG
CTATTGATCAGATTACCGGGAAGCTGAATAGACTGATTGAAAAGACCAACCAGCAGTTCGAGCTG
ATCGATAACGAGTTTAACGAGGTCGAAAAGCAGATAGGAAATGTAATCAGGTGGACACGCGATTC
TATCACCGAAGTCTGGTCCTACAATGCCGAGCTGCTGGTTGCAATGGAGAACCAACACACTATCGA
CCTTGCCGATTCCGAAATGGACAAACTGTATGAACGCGTGAAGCGGCAGCTCAGGGAAAACGCTG
AGGAAGACGGTACCGGCTGTTTCGAAATTTTCCACAAGTGCGATGATGACTGTATGGCCAGCATCA
GGCACAACACCTACGATCATTCCAAGTATAGAGAGGAGGCCATGCAGAACCGGATTCAGATCGAT
CCAGTGAAACTGTCATCCGGCTACAAGGACGTGCTCGAGACCGGCGGCGGATACATTCCAGAGGC
CCCCAGAGACGGACAGGCTTATGTGAGAAAGGACGGAGAGTGGGTGCTGCTGAGTACATTTCTG
SEQ ID NO:39 (Amino acid sequence of 4M2e-HA2-collagen (M4HC))* 
SLLTEVETPIRNEWGCRCNGSSDPAAASLLTEVETPIRNEWGCRCNDSSDPAAASLLTEVETPTRTGWE
CNCSGSSEPAAASLLTEVETPTRNEWECKCSDSSDPKTACTSAPGTPGLFGAIAGFIENGWEGLIDGWY
GFRHQNAQGEGTAADYKSTQSAIDQITGKLNRLIEKTNQQFELIDNEFNEVEKQIGNVIRWTRDSITEV
WSYNAELLVAMENQHTIDLADSEMDKLYERVKRQLRENAEEDGTGCFEIFHKCDDDCMASIRHNTYD
HSKYREEAMQNRIQIDPVKLSSGYKDVDKIREVPEGWLIFVAEREELYVRVRNGFRKVLLEARTALPR
GTGNE
SEQ ID NO:40 (Nucleotide sequence of 4M2e-HA2-collagen (M4HC)) 
TCCCTCCTGACCGAGGTGGAGACACCTATCCGGAACGAGTGGGGGTGTAGATGCAACGGCAGCAG
CGACCCAGCTGCCGCAAGCCTCCTCACAGAGGTGGAGACACCAATCAGGAACGAGTGGGGATGCA
GATGCAACGACTCTAGCGACCCAGCTGCCGCAAGTCTCCTCACAGAGGTGGAGACACCTACCAGG
ACCGGCTGGGAGTGCAACTGCTCTGGCAGCTCTGAGCCAGCTGCCGCTTCTCTCCTCACAGAGGTG
GAGACACCTACAAGAAACGAGTGGGAGTGCAAGTGCTCCGACTCTTCCGACCCAAAGACCGCTTG
TACAAGCGCCCCAGGCACACCTGGCCTTTTTGGAGCAATCGCTGGATTCATTGAAAATGGGTGGGA
GGGCCTCATCGACGGGTGGTACGGATTCAGGCACCAAAACGCCCAGGGAGAAGGGACCGCAGCT
GACTACAAGTCAACACAGTCAGCTATTGATCAGATTACCGGGAAGCTGAATAGACTGATTGAAAA
GACCAACCAGCAGTTCGAGCTGATCGATAACGAGTTTAACGAGGTCGAAAAGCAGATAGGAAATG
TAATCAGGTGGACACGCGATTCTATCACCGAAGTCTGGTCCTACAATGCCGAGCTGCTGGTTGCAA
TGGAGAACCAACACACTATCGACCTTGCCGATTCCGAAATGGACAAACTGTATGAACGCGTGAAG
CGGCAGCTCAGGGAAAACGCTGAGGAAGACGGTACCGGCTGTTTCGAAATTTTCCACAAGTGCGA
TGATGACTGTATGGCCAGCATCAGGCACAACACCTACGATCATTCCAAGTATAGAGAGGAGGCCA
TGCAGAACCGGATTCAGATCGATCCAGTGAAACTGTCATCCGGCTACAAGGACGTGGACAAGATC
AGGGAGGTGCCTGAGGGCTGGCTCATCTTCGTGGCCGAGCGGGAGGAGCTGTACGTGCGGGTGAG
AAACGGCTTCAGAAAGGTGCTCTTAGAAGCCCGGACAGCCCTGCCACGGGGCACAGGCAACGAG
SEQ ID NO:41 (IRES nucleotide sequence) GAGGGCCCGGAAACCTGGCCCTGTCTTCTTGACGAGCATTCCTAGGGGTCTTTCCCCTCTCGCCAA 
AGGAATGCAAGGTCTGTTGAATGTCGTGAAGGAAGCAGTTCCTCTGGAAGCTTCTTGAAGACAAA
CAACGTCTGTAGCGACCCTTTGCAGGCAGCGGAACCCCCCACCTGGCGACAGGTGCCTCTGCGGCC
AAAAGCCACGTGTATAAGATACACCTGCAAAGGCGGCACAACCCCAGTGCCACGTTGTGAGTTGG
ATAGTTGTGGAAAGAGTCAAATGGCTCTCCTCAAGCGTATTCAACAAGGGGCTGAAGGATGCCCA
GAAGGTACCCCATTGTATGGGATCTGATCTGGGGCCTCGGTGCACATGCTTTACATGTGTTTAGTC
GAGGTTAAAAAAACGTCTAGGCCCCCCGAACCACGGGGACGTGGTTTTCCTTTGAAAAACACGAT
GATAAGCCACC
SEQ ID NO:42 (Nucleotide sequence of 4M2e- HA2-foldon-IRES-T-DHFR)* 
TCCCTCCTGACCGAGGTGGAGACACCTATCCGGAACGAGTGGGGGTGTAGATGCAACGGCAGCAG
CGACCCAGCTGCCGCAAGCCTCCTCACAGAGGTGGAGACACCAATCAGGAACGAGTGGGGATGCA
GATGCAACGACTCTAGCGACCCAGCTGCCGCAAGTCTCCTCACAGAGGTGGAGACACCTACCAGG
ACCGGCTGGGAGTGCAACTGCTCTGGCAGCTCTGAGCCAGCTGCCGCTTCTCTCCTCACAGAGGTG
GAGACACCTACAAGAAACGAGTGGGAGTGCAAGTGCTCCGACTCTTCCGACCCAAGCGCCCCAGG
CACACCTGGCCTTTTTGGAGCAATCGCTGGATTCATTGAAAATGGGTGGGAGGGCCTCATCGACGG
GTGGTACGGATTCAGGCACCAAAACGCCCAGGGAGAAGGGACCGCAGCTGACTACAAGTCAACA
CAGTCAGCTATTGATCAGATTACCGGGAAGCTGAATAGACTGATTGAAAAGACCAACCAGCAGTT
CGAGCTGATCGATAACGAGTTTAACGAGGTCGAAAAGCAGATAGGAAATGTAATCAATTGGACAC
GCGATTCTATCACCGAAGTCTGGTCCTACAATGCCGAGCTGCTGGTTGCAATGGAGAACCAACACA
CTATCGACCTTGCCGATTCCGAAATGGACAAACTGTATGAACGCGTGAAGCGGCAGCTCAGGGAA
AACGCTGAGGAAGACGGTACCGGCTGTTTCGAAATTTTCCACAAGTGCGATGATGACTGTATGGCC
AGCATCAGGAATAACACCTACGATCATTCCAAGTATAGAGAGGAGGCCATGCAGAACCGGATTCA
GATCGATCCAGTGAAACTGTCATCCGGCTACAAGGACGTGCTCGAGACCGGCGGCGGATACATTC
CAGAGGCCCCCAGAGACGGACAGGCTTATGTGAGAAAGGACGGAGAGTGGGTGCTGCTGAGTAC
ATTTCTGGAGGGCCCGGAAACCTGGCCCTGTCTTCTTGACGAGCATTCCTAGGGGTCTTTCCCCTCT
CGCCAAAGGAATGCAAGGTCTGTTGAATGTCGTGAAGGAAGCAGTTCCTCTGGAAGCTTCTTGAA
GACAAACAACGTCTGTAGCGACCCTTTGCAGGCAGCGGAACCCCCCACCTGGCGACAGGTGCCTC
TGCGGCCAAAAGCCACGTGTATAAGATACACCTGCAAAGGCGGCACAACCCCAGTGCCACGTTGT
GAGTTGGATAGTTGTGGAAAGAGTCAAATGGCTCTCCTCAAGCGTATTCAACAAGGGGCTGAAGG
ATGCCCAGAAGGTACCCCATTGTATGGGATCTGATCTGGGGCCTCGGTGCACATGCTTTACATGTG
TTTAGTCGAGGTTAAAAAAACGTCTAGGCCCCCCGAACCACGGGGACGTGGTTTTCCTTTGAAAAA
CACGATGATAAGCCACCATGGACGTGAACCCCACCCTGCTGTTTCTGAAGGTGCCCGCTCAGAACG
CCATCTCCACCACATTCCCCTACACCGGCGACCCTCCTTACTCCCATGGCACAGGAACCGGCTACA
CCATGGACACCGTGAACAGAACCCACCAGTACAGCGAGAAAGGCAAATGGACCACCAACACCGA
AACCGGCGCCCCTCAGCTGAACCCTATCGATGGACCCCTGCCCGAAGACAACGAACCCTCAGGAT
ACGCCCAGCACTTCCAGAGAAAAAGAAGAGTGAGAGACAACATGACCAAAAAGATGGTGACCCA
GAGAACCATCGGCAAGAAGAAACAGAGACTGAACAAGAGAGGATACCTGATCAGAGCCCTGACC
CTGAACACCATGACCAAGGACGCCGAGAGAGGCAAGCTGAAGAGAAGAGCCATCGCCACCCCCG
GAATGCAGATCAGAGGATTCGTGTACTTCGTGGAGACCCTGGCCAGAAGCATCTGCGAAAAGCTG
GAGCAGAGCGGACTGCCAGTGGGAGGAAACGAAAAAAAGGCCAAGCTGGCCAACGTGGTGAGAA
AGATGATGACCAACAGCCAGGACACCGAGATCAGCTTCACCATCACCGGCGACAACACCAAGTGG
AACGAGAACCAGAACCCCAGAATGTTCCTGGCCATGATCACCTACATCACCAAGAACCAGCCCGA
GTGGTTCAGAAACATCCTGAGCATCGCCCCCATCATGTTCAGCAACAAGATGGCCAGACTGGGCA
AGGGCTACATGTTCGAGAGCAAGAGAATGAAGCTGAGAACCCAGATCCCCGCCGAGATGCTGGCC
AGCATCGACCTGAAGTACTTCAACGAGAGCACCAAGAAGAAGATCGAGAAGATCAGACCCCTGCT
GATCGACGGCACCGCCAGCCTGAGCCCCGGCATGATGATGGGCATGTTCAACATGCTGAGCACCG
TGCTGGGCGTGAGCATCCTGAACCTGGGCCAGAAGAAGTACACCAAGACCACCTACTGGTGGGAC
GGCCTGCAGAGCAGCGACGACTTCGCCCTGATCGTGAACGCCCCCAACCACGAGGGCATCCAGGC
CGGCGTGGACAGATTCTACAGAACCTGCAAGCTGGTGGGCATCAACATGAGCAAGAAGAAGAGCT
ACATCAACAAGACCGGCACCTTCGAGTTCACCAGCTTCTTCTACAGATACGGCTTCGTGGCCAACT
TCAGCATGGAGCTGCCCAGCTTCGGCGTGAGCGGCGTGAACGAGAGCGCCGACATGAGCATCGGC
GTGACCGTGATCAAGAACAACATGATCAACAACGACCTGGGCCCCGCCACCGCCCAGATGGCCCT
GCAGCTGTTCATCAAGGACTACAGATACACCTACAGATGCCACAGAGGCGACACCCAGATCCAGA
CCAGAAGAAGCTTCGAGCTGAAGAAGCTGTGGGACCAGACCCAGAGCAAGGCCGGCCTGCTGGTG
AGCGACGGCGGCCCCAACCTGTACAACATCAGAAACCTGCACATCCCCGAGGTGTGCCTGAAGTG
GGAGCTGATGGACGAGGACTACAGAGGCAGACTGTGCAACCCCCTGAACCCCTTCGTGAGCCACA
AGGAGATCGAGAGCGTGAACAACGCCGTGGTGATGCCCGCCCACGGCCCCGCCAAGAGCATGGA
GTACGACGCCGTGGCCACCACCCACAGCTGGATTCCTAAGAGAAACAGAAGCATCCTGAACACCA
GCCAGAGAGGCATCCTGGAGGACGAGCAGATGTACCAGAAGTGCTGCAACCTGTTCGAGAAGTTC
 
TTCCCCAGCAGCAGCTACAGAAGACCCGTGGGCATCAGCAGCATGGTGGAGGCCATGGTGAGCAG
AGCCAGAATCGACGCCAGAATCGACTTCGAGAGCGGCAGAATCAAGAAGGAGGAGTTCAGCGAG
ATCATGAAGATCTGCAGCACCATCGAGGAGCTGAGAAGACAGAAGGGCGGCGGCGGCAGCGGCG
GCGGCGGCAGCATGAGAAGAAACTACTTCACCGCCGAGGTGAGCCACTGCAGAGCCACCGAGTAC
ATCATGAAGGGCGTGTACATCAACACCGCCCTGCTGAACGCCAGCTGCGCCGCCATGGACGACTT
CCAGCTGATCCCCATGATCAGCAAGTGCAGAACCAAGGAGGGCAGAAGAAAGACCAACCTGTAC
GGCTTCATCATCAAGGGCAGAAGCCACCTGAGAAACGACACCGACGTGGTGAACTTCGTGAGCAT
GGAGTTCAGCCTGACCGACCCCAGACTGGAGATGTTCCTGTACGTGAGAACCAACGGCACCAGCA
AGATCAAGATGAAGTGGGGCATGGAGATGAGAAGATGCCTGCTGCAGAGCCTGCAGCAGATCGA
GAGCATGATCGAGGCCGAGAGCAGCGTGAAGGAGAAGGACATGACCAAGGAGTTCTTCGAGAAC
AAGAGCGAGACCTGGCCCATCGGCGAGAGCCCCAAGGGCGTGGAGGAGGGCAGCATCGGCAAGG
TGTGCAGAACCCTGCTGGCCAAGAGCGTGTTCAACTTCGACCTGGGCGGCCTGTACGAGGCCATCG
AGGAGTGCCTGATCAACGACCCCTGGGTGCTGCTGAACGCCAGCTGGTTCAACAGCTTCCTGACCC
ACGCCCTGAAGGGCAGCGGCGGCAGCGGCATGACCAAGATCACCCTGAGCCCCCAGAACTTCAGA
ATCCAGAAGCAGGAGACCACCCTGCTGAAGGAGAAGAGCACCGAGAAGAACAGCCTGGCCAAGA
GCATCCTGGCCGTGAAGAACCACTTCATCGAGCTGAGAAGCAAGCTGAGCGAGAGATTCATCAGC
CACAAGAACACCGAGAGCAGCGCCACCCACTTCCACAGAGGCAGCGCCAGCGAGGGCAGAGCCG
TGCTGACCAACAAGGTGGTGAAGGACTTCATGCTGCAGACCCTGAACGACATCGACATCAGAGGC
AGCGCCGCGGCCGCCACCATGATCAGCCTGATCGCCGCCCTGGCCGTGGACTACGTGCTGGGCAT
GGAGAACGCCATGCCCTGGAACCTGCCCGCCGACCTGGCCTGGTTCAAGCGGAACACCCTGAACA
AGCCCGTGATCATGGGCAGACACACCTGGGAGTCCATCGGCAGACCCCTGCCCGGCAGAAAGAAC
ATCATCCTATCGTCCCAGCCCTCCACCGACGACCGGGTGACCTGGGTGAAGAGCGTGGACGAGGC
CATCGCCGCCTGCGGCGACGTGCCCGAGATCATGGTGATCGGCGGCGGCAGAGTGATCGAGCAGT
TCCTGCCCAAGGCCCAGAAGCTGTACCTGACCCACCTGGACGCCGAGGTGGAGGGCGACACCCAC
TTCCCCGACTACGAGCCCGACGACTGGGAGTCCGTGTTCAGCGAGTTCCACGACGCCGACGCCCA
GAACAGCCACTCCTACTGCTTCGAGATCCTGGAGAGAAGGGGCGGCGGCGGCTCT
SEQ ID NO:43 (DHFR amino acid sequence) 
MISLIAALAVDYVLGMENAMPWNLPADLAWFKRNTLNKPVIMGRHTWESIGRPLPGRKNIILSSQPST
DDRVTWVKSVDEAIAACGDVPEIMVIGGGRVIEQFLPKAQKLYLTHLDAEVEGDTHFPDYEPDDWESV
FSEFHDADAQNSHSYCFEILERRGGGGS
SEQ ID NO:44 (DHFR nucleotide sequence) 
ATGATCAGCCTGATCGCCGCCCTGGCCGTGGACTACGTGCTGGGCATGGAGAACGCCATGCCCTG
GAACCTGCCCGCCGACCTGGCCTGGTTCAAGCGGAACACCCTGAACAAGCCCGTGATCATGGGCA
GACACACCTGGGAGTCCATCGGCAGACCCCTGCCCGGCAGAAAGAACATCATCCTATCGTCCCAG
CCCTCCACCGACGACCGGGTGACCTGGGTGAAGAGCGTGGACGAGGCCATCGCCGCCTGCGGCGA
CGTGCCCGAGATCATGGTGATCGGCGGCGGCAGAGTGATCGAGCAGTTCCTGCCCAAGGCCCAGA
AGCTGTACCTGACCCACCTGGACGCCGAGGTGGAGGGCGACACCCACTTCCCCGACTACGAGCCC
GACGACTGGGAGTCCGTGTTCAGCGAGTTCCACGACGCCGACGCCCAGAACAGCCACTCCTACTG
CTTCGAGATCCTGGAGAGAAGGGGCGGCGGCGGCTCT
SEQ ID NO: 45 (Peptide linker)
   GGGGS
SEQ ID NO: 46 (Peptide linker)
   GSAGSAAGSGEF
SEQ ID NO: 47 (Peptide linker)
   EAAAK
SEQ ID NO: 48 (Peptide linker)
   EFPKPSTPPGSSGGAP
SEQ ID NO: 49 (Peptide linker)
   KESGSVSSEQLAQFRSLD
SEQ ID NO: 50 (Peptide linker)
   EGKSSGSGSESKST
**SEQ** ID NO: 51 (T cell antigen 1) 
MERIKELRNLMSQSRTREILTKTTVDHMAIIKKYTSGKWMMAMKYPITADKRITEMIPERNEQGQTLW
SKMNDAGSDRVMVSPLAVTWWNRNGPVTSTVHYPKVYKTYFEKVERLKHGTFGPVHFRNQVKQLTI
TKEKKEELQDCKISPLMVAYMLERELVRKTRFLPVAGGTSSVYIEVLIVRRAAVSADPLASLLEMCHSG
LRISSSFSFGGFTFKRTSGSSVKKEEEVLTGNLQTLKIRIVSGRDEQSIAEAIIVAMVFSPMHQLLRHFQK
DAKVLFQNWGIEHIDNVMGMIGILPDMTPSTEMSVSIDRFLRVRDQRGNVLLSPEEVSETQGTEKLTIT
YSSSMMWEINGPESVLVNTYQWIIRNWEAVKIQWSQNPTMLYNKMEFEPFQSLVPKAIRSQYSGFVRT
LFQQMRDVLGTFDTTQIIKLLPFAAAPPKQSRMQFSSLTVNVRGSGMRILVRGNSPVFNYNKTTKRLTV
LGKDAGTLTEDPDEGTSGVESAVLRGFLILGKEDRRYGPALSINELSVMKRKRDSSILTDSQTATKRIR
MAINGSGGSGMASQGTKRSYEQMETDGERQNATEIRASVGRMIGGIGRFYIQMCTELKLSDYEGRLIQ
NSLTIERMVLSAFDERRNKYLEEHPSAGKDPKKTGGPIYRRVDGKWMRELVLYDKEEIRRIWRQANNG
EDATAGLTHIMIWHSNLNDATYQRTRALVRTGMDPRMCSLMQGSTLPRRSGAAGAAVKGVGTMVM
ELIRMIKRGINDRNFWRGENGRKTRVAYERMCNILKGKFQTAAQRAMMDQVRESRNPGNAEIEDLIFL
ARSALILRGSVAHKSCLPACVYGPAVASGYDFEKEGYSLVGIDPFKLLQNSQVYSLIRPNENPAHKSQL
VWMACHSAAFEDLRVSSFIRGTKVIPRGKLSTRGVQIASNENMDTMDSSTLELRSRYWAIRTRSGGNT
NQQRASAGQISVQPTFSVQRNLPFEKSTVMAAFTGNTEGRTSDMRAEIIRMMESAKPEEVSFQGRGVFE
LSDEKATNPIVPSFDMSNEGSYFFGDNAEEYDNGGGGSGGGGSGGGGSMSLLTEVETPIRNEWGCRCN
DSSDPLVVAANIIGILHLILWILDRLFFKCIYRLFKHGLKRGPSTEGVPESMREEYRKEQQNAVDADDSH
FVSIELE
SEQ ID NO: 52 (T cell antigen 2)

MDVNPTLLFLKVPAQNAISTTFPYTGDPPYSHGTGTGYTMDTVNRTHQYSEKGKWTTNTETGAPQLNP
IDGPLPEDNEPSGYAQHFQRKRRVRDNMTKKMVTQRTIGKKKQRLNKRGYLIRALTLNTMTKDAERG
KLKRRAIATPGMQIRGFVYFVETLARSICEKLEQSGLPVGGNEKKAKLANVVRKMMTNSQDTEISFTIT
GDNTKWNENQNPRMFLAMITYITKNQPEWFRNILSIAPIMFSNKMARLGKGYMFESKRMKLRTQIPAE
MLASIDLKYFNESTKKKIEKIRPLLIDGTASLSPGMMMGMFNMLSTVLGVSILNLGQKKYTKTTYWWD
GLQSSDDFALIVNAPNHEGIQAGVDRFYRTCKLVGINMSKKKSYINKTGTFEFTSFFYRYGFVANFSME
LPSFGVSGVNESADMSIGVTVIKNNMINNDLGPATAQMALQLFIKDYRYTYRCHRGDTQIQTRRSFELK
KLWDQTQSKAGLLVSDGGPNLYNIRNLHIPEVCLKWELMDEDYRGRLCNPLNPFVSHKEIESVNNAVV
MPAHGPAKSMEYDAVATTHSWIPKRNRSILNTSQRGILEDEQMYQKCCNLFEKFFPSSSYRRPVGISSM
VEAMVSRARIDARIDFESGRIKKEEFSEIMKICSTIEELRRQKGGGGSGGGGSGGGGSMRRNYFTAEVS
HCRATEYIMKGVYINTALLNASCAAMDDFQLIPMISKCRTKEGRRKTNLYGFIIKGRSHLRNDTDVVNF
VSMEFSLTDPRLEMFLYVRTNGTSKIKMKWGMEMRRCLLQSLQQIESMIEAESSVKEKDMTKEFFENK
SETWPIGESPKGVEEGSIGKVCRTLLAKSVFNFDLGGLYEAIEECLINDPWVLLNASWFNSFLTHALKGS
GGSGMSLLTEVETYVLSIVPSGPLKAEIAQRLEDVFAGKNTDLEALMEWLKTRPILSPLTKGILGFVFTL
TVPSERGLQRRRFVQNALNGNGDPNNMDRAVKLYRKLKREITFHGAKEIALSYSAGALASCMGLIYNR
MGAVTTEVAFGLVCATCEQIADSQHRSHRQMVTTTNPLIRHENRMVLASTTAKAMEQMAGSSEQAAE
AMEVASQARQMVQAMRAIGTHPSSSTGLKDDLLENLQAYQKRMGVQMQRFK

## Claims

1. A nucleic acid molecule encoding an immunogenic peptide, wherein the immunogenic peptide comprises:
(a) a tandem M2 protein extracellular region (M2e) peptide segment;
(b) a hemagglutinin protein stem extracellular domain (HA2) peptide segment derived from an H7N9 influenza virus subtype;
(c) a multimerized motif peptide segment;
(d) optionally, one or more linker peptide segments independently located between the peptide segments;
the nucleic acid molecule comprises nucleic acids encoding each of the peptide segments.

2. The nucleic acid molecule of claim 1, wherein,
the M2e peptide segment is derived from one or more influenza virus strains selected from the group consisting of H1N1, H2N2, H3N2, H3N8, H5N1, H5N2, H5N6, H7N1, H7N2, H7N3, H7N7, H7N9, H9N2, H10N8, and H11N2 subtypes; and/or
the M2e peptide segment is derived from influenza virus strains whose hosts are animals selected from the group consisting of: mammalian (e.g., human, non-human primates, swine, horse, cattle, sheep, bat, sea lion, seal, rat, mouse, ferret), avian (e.g., domestic poultry or wild birds), or zoonotic (e.g., human-animal, human-avian, or swine-avian) influenza virus strains; and/or
each of the M2e regions in the M2e peptide segment is derived from the same or different sources; and/or
the M2e peptide segment comprises 2-12 tandem M2e regions, preferably 4-8 tandem M2e regions, such as 4, 5, 6, 7, 8, 9, 10, 11, or 12 tandem M2e regions; and/or
each of the M2e regions in the M2e peptide is directly linked or linked via a linker, for example, the linker is independently selected from flexible linkers, rigid linkers, and cleavable linkers, such as AAA, (G4S)ₙ, e.g., (G4S)₃, GSAGSAAGSGEF, (Gly)ₙ, (EAAAK)ₙ, EFPKPSTPPGSSGGAP, KESGSVSSEQLAQFRSLD, EGKSSGSGSESKST, where n is an integer between 2 and 10, e.g., n=3; and/or
the M2e region has the amino acid sequence set forth in SEQ ID NO: 13, SEQ ID NO: 15, SEQ ID NO: 17, or SEQ ID NO: 19, or an amino acid sequence having at least 80% sequence identity with said amino acid sequences; and/or
the nucleic acid encoding the M2e region has the nucleotide sequences set forth in SEQ ID NO: 14, SEQ ID NO: 16, SEQ ID NO: 18, and SEQ ID NO: 20, or a nucleotide sequence having at least 80% sequence identity with said nucleotide sequences, e.g., sequences obtained by codon optimization or substitution of one or more nucleotides from the aforementioned nucleotide sequences; and/or
the M2e peptide segment has the amino acid sequence set forth in SEQ ID NO: 21 or SEQ ID NO: 23, or an amino acid sequence having at least 80% sequence identity with said amino acid sequence; and/or
the nucleic acid encoding the M2e peptide segment has the nucleotide sequence set forth in SEQ ID NO: 22 or SEQ ID NO: 24, or a nucleotide sequence having at least 80% sequence identity with said nucleotide sequence, e.g., a sequence obtained by codon optimization or substitution of one or more nucleotides of the aforementioned nucleotide sequence.

3. The nucleic acid molecule of claim 1, wherein the HA2 peptide is derived from an H7N9 subtype strain, for example from A/Shanghai/02/2013 strain or A/Anhui/01/2013 strain; and/or
the HA2 peptide segment comprises one or more mutations compared to the native HA2 peptide segment, such as an N82 mutation at amino acid position 82, preferably N82R, N82K, N82D, or N82E; and an N154 mutation at position 154, preferably N154H; and/or
the HA2 peptide segment is linked to a partial fragment of HA1, for example, comprising an HA2 peptide segment that anchors the HA protein to the stem of the viral lipid envelope, and a partial HA1 peptide segment; and/or
the HA2 peptide segment has the amino acid sequence set forth in SEQ ID NO: 1, or an amino acid sequence having at least 80% sequence identity with said amino acid sequence; and/or
the nucleic acid encoding the HA2 peptide segment has the nucleotide sequence set forth in SEQ ID NO: 2, or a nucleotide sequence having at least 80% sequence identity with said nucleotide sequence, e.g., a sequence obtained by codon optimization or substitution of one or more nucleotides of the aforementioned nucleotide sequence.

4. The nucleic acid molecule of claim 1, wherein the multimerized motif peptide segment is selected from collagen, the trimerized motif Foldon of T4 phage fibrin, the tetrabrachion helical peptide, and GCN4; and/or
the multimerized motif peptide segment has the amino acid sequences set forth in SEQ ID NO: 3, SEQ ID NO: 5, SEQ ID NO: 7, and SEQ ID NO: 9, or an amino acid sequence having at least 80% sequence identity with said amino acid sequences; and/or
the nucleic acid encoding the multimerized motif peptide segment has the nucleotide sequence set forth in SEQ ID NO: 4, SEQ ID NO: 6, SEQ ID NO: 8, and SEQ ID NO: 10, or a nucleotide sequence having at least 80% sequence identity with said nucleotide sequence, e.g., a sequence obtained by codon optimization or substitution of one or more nucleotides from the aforementioned nucleotide sequence.

5. The nucleic acid molecule of claim 1, wherein the immunogenic peptide further comprises (e) one or more other peptide segments,
for example, the other peptide segments comprise one or more peptide segments selected from the group consisting of T-cell antigen peptides, transferrin, FF peptides, cyclic peptide nanotubes, and IL-21;
for example, other peptide segments comprise a T-cell antigen peptide segment having the amino acid sequence set forth in SEQ ID NOs: 11, 51, or 52, or an amino acid sequence having at least 80% sequence identity with said amino acid sequence; and/or
the nucleic acid encoding the T-cell antigen peptide segment has the nucleotide sequence set forth in SEQ ID NO: 12 or is a nucleic acid encoding the polypeptide set forth in SEQ ID NO: 51 or 52, or a nucleotide sequence having at least 80% sequence identity with said nucleic acid, e.g., a sequence obtained by codon optimization or substitution of one or more nucleotides of the aforementioned nucleotide sequence; and/or
the T-cell antigen peptide is linked to a linking element, for example, the linking element is selected from IRES, T2A, P2A; and/or
the T-cell antigen peptide is further linked to a degron, such as the degron DHFR.

6. The nucleic acid molecule of claim 1, wherein the peptide segments of the immunogenic peptide are independently linked directly or via a linker, for example, the linker is independently selected from: flexible linkers, rigid linkers and cleavable linkers, such as AAA, (G4S)ₙ (e.g. (G4S)₃), GSAGSAAGSGEF, (Gly)ₙ, (EAAAK)ₙ, EFPKPSTPPGSSGGAP, KESGSVSSEQLAQFRSLD, EGKSSGSGSESKST, where n is an integer between 2 and 10 (e.g., n=3); and/or
the immunogenic peptide has the amino acid sequences set forth in SEQ ID NO: 25, SEQ ID NO: 27, SEQ ID NO: 33, and SEQ ID NO: 39, or an amino acid sequence having at least 80% sequence identity with said amino acid sequences; and/or
the nucleic acid encoding the immunogenic peptide has the nucleotide sequence set forth in SEQ ID NO: 26, SEQ ID NO: 28, SEQ ID NO: 34, SEQ ID NO: 40, SEQ ID NO: 42, or a nucleotide sequence having at least 80% sequence identity with said nucleotide sequence, e.g., a sequence obtained by codon optimization or substitution of one or more nucleotides of the aforementioned nucleotide sequence.

7. The nucleic acid molecule of claim 1, wherein the nucleic acid molecule is a DNA molecule or an RNA molecule,
for example, the nucleic acid molecule is an mRNA molecule, such as a self-amplifying mRNA (SAM) molecule or a non-replicating mRNA molecule, preferably a SAM molecule; wherein, when the mRNA molecule is a non-replicating mRNA molecule, it also comprises a 5'-cap structure and a 3'-poly(A) tail.

8. An immunogenic peptide, characterized as defined in any one of claims 1-6, or encoded by a nucleic acid molecule of any one of claims 1-7.

9. A vector or host cell comprising the nucleic acid molecule according to any one of claims 1-7,
for example, the vector is selected from mRNA vectors, DNA plasmid vectors, recombinant viral vectors, and recombinant bacterial vectors; wherein the mRNA vector is selected from linear, circular, and self-replicating vectors; and the recombinant viral vector is selected from poxviruses (e.g., Tian Tan strain, North American vaccine strain, Wyeth derivative strain, Listeria strain, Ankara derivative strain, Copenhagen strain, and New York strain), adenoviruses (e.g., adenovirus types 5, 11, 26, 35, 63, and 68), adeno-associated viruses, herpes simplex virus, measles virus, enterovirus, reovirus, rhabdovirus, flavivirus, influenza virus, parainfluenza virus, respiratory syncytial virus, and poliovirus vectors;
for example, the host cells are mammalian or insect cells, such as HEK293, HeLa, K562, CHO, NS0, SP2/0, PER.C6, Vero, RD, BHK, HT 1080, A549, Cos-7, ARPE-19, and MRC-5 cells; High Five, Sf9, Se301, SeIZD2109, SeUCR1, Sf9, Sf900+, Sf21, BTI-TN-5B1-4, MG-1, Tn368, HzAm1, BM-N, Ha2302, Hz2E5, and Ao38.

10. An influenza vaccine comprising
(i) one or more nucleic acid molecules of any one of claims 1-7, the immunogenic peptide of claim 8, or the vector of claim 9;
(ii) an immunologically acceptable carrier, delivery system or adjuvant.

11. The influenza vaccine of claim 10, wherein,
the influenza vaccine is an mRNA vaccine, a DNA vaccine, a viral vector vaccine, a recombinant protein vaccine; and/or
the carrier or delivery system is selected from lipid delivery systems, lipid-like delivery systems, polymer delivery systems, or combinations thereof, such as loaded on lipid nanoparticles (e.g., a combination of cationic lipids, structural lipids, accessory lipids, and stabilizing lipids), polyurethane (PAA), poly-β-amino ester (PBAE), polyethyleneimine (PEI), lipid-encapsulated polymer micelles; and/or
the vaccine also comprises an adjuvant or is used in combination with an adjuvant, for example, the adjuvant is selected from aluminum adjuvant, cholera toxin and subunits thereof, oligodeoxynucleotides, manganese ion adjuvant, colloidal manganese adjuvant, Freund's adjuvant, MF59 adjuvant, QS-21 adjuvant, Poly I:C and other TLR ligands, GM-CSF, IL-2, IL-3, IL-7, IL-11, IL-12, IL-18, IL-21; and/or
the vaccine is suitable for one or more administration or delivery methods selected from the group consisting of respiratory nebulization, intranasal drops, oral administration, direct injection (e.g., intravenous injection, subcutaneous injection, intradermal injection, intramuscular injection), mucosal administration; and/or
the vaccine is in a form suitable for administration of two or more drugs or vaccines in combination, such as combined or sequential administration.

12. Use of the nucleic acid molecule of any one of claims 1-7, the immunogenic peptide of claim 8, the vector of claim 9, and/or the influenza vaccine of any one of claims 10-11 in the manufacture of a product for the prevention and/or treatment of influenza.
